(19) Europäisches Patentamt
European Patent Office
Office européen des brevets

(11) **EP 2 766 456 B1**

(12) **EUROPÄISCHE PATENTSCHRIFT**

(45) Veröffentlichungstag und Bekanntmachung des Hinweises auf die Patenterteilung:
**10.05.2017 Patentblatt 2017/19**

(21) Anmeldenummer: **12774998.4**

(22) Anmeldetag: **10.10.2012**

(51) Int Cl.:
***C10G 3/00*** *(2006.01)*

(86) Internationale Anmeldenummer:
**PCT/EP2012/070055**

(87) Internationale Veröffentlichungsnummer:
**WO 2013/053754 (18.04.2013 Gazette 2013/16)**

(54) **VERFAHREN ZUR GEWINNUNG VON KOHLENWASSERSTOFFEN MIT MITTLEREN KETTENLÄNGEN**

METHOD FOR EXTRACTING HYDROCARBONS WITH MEDIUM CHAIN LENGTHS

PROCÉDÉ DE PRODUCTION D'HYDROCARBURES AYANT DES LONGUEURS DE CHAÎNE MOYENNES

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **10.10.2011 DE 102011115377**

(43) Veröffentlichungstag der Anmeldung:
**20.08.2014 Patentblatt 2014/34**

(73) Patentinhaber: **Fraunhofer-Gesellschaft zur Förderung der
angewandten Forschung e.V.
80686 München (DE)**

(72) Erfinder:
• **UNGER, Christoph**
**46485 Wesel (DE)**
• **MENNE, Andreas**
**45470 Mülheim a.d. Ruhr (DE)**
• **KRAFT, Axel**
**45759 Oer-Erkenschwick (DE)**
• **HEIL, Volker**
**46145 Oberhausen (DE)**

(56) Entgegenhaltungen:
EP-A1- 1 724 325    DE-A1- 10 327 059
DE-A1-102008 049 778    US-A- 1 960 951
US-A- 1 991 955    US-A1- 2008 034 645
US-A1- 2010 296 997

• FU J ET AL: "Activated carbons for hydrothermal decarboxylation of fatty acids", ACS CATALYSIS, AMERICAN CHEMICAL SOCIETY, US, Bd. 1, Nr. 3, 1. Januar 2011 (2011-01-01), Seiten 227-231, XP008160266, ISSN: 2155-5435, DOI: 10.1021/CS1001306 [gefunden am 2011-02-15]
• Gunter Festel ET AL: "Decarboxylation of fatty oils and fatty acids - a novel route towards bio-jet fuel", , 4. Juni 2012 (2012-06-04), XP055054303, Toulouse, France Gefunden im Internet: URL:http://www.greasoline.com/Downloads/Poster_RRB8_greasoline.pdf [gefunden am 2013-02-22]
• SUDARYANTO Y ET AL: "High surface area activated carbon prepared from cassava peel by chemical activation", BIORESOURCE TECHNOLOGY, ELSEVIER BV, GB, vol. 97, no. 5, 1 March 2006 (2006-03-01), pages 734-739, XP027965419, ISSN: 0960-8524 [retrieved on 2006-03-01]

**Beschreibung**

Die Anmeldung betrifft ein Verfahren zur Gewinnung von Kohlenwasserstoffen mit mittleren Kettenlängen, d.h. Kohlenwasserstoffen mit 8 bis 16 Kohlenstoffatomen. Bei diesem Verfahren werden ungesättigte sauerstoffhaltige Kohlenwasserstoffverbindungen als Ausgangsmaterial eingesetzt und mit einem porösen Katalysator auf Kohlenstoff-Basis bei erhöhter Temperatur in Kontakt gebracht. Die Anmeldung betrifft ferner die Verwendung des mit diesem Verfahren erhaltenen Kohlenwasserstoffgemischs bzw. damit erhaltener reiner Kohlenwasserstoffe zur Herstellung von Flugzeugtreibstoff. Kohlenwasserstoffe mit mittleren Kettenlängen sind einerseits wesentliche Rohstoffe für die chemische Industrie, andererseits besteht auch ein Bedarf von insbesondere sauerstofffreien Kohlenwasserstoffen mit mittleren Kettenlängen als wichtiger Baustein für Kerosin zur Anwendung in der Luftfahrt, etwa als "Drop-in-Aviation-Fuel" und hier insbesondere in Form von Bio-Kerosin. Bio-Kerosin wird gemäß dem Stand der Technik beispielsweise durch Hydrieren biobasierter Öle gewonnen. Nach dem Stand der Technik ist ferner bekannt, dass durch Umsetzung von Fetten, Ölen bzw. Fettsäuren an porösen Katalysatoren entweder flüssige Kohlenwasserstoffe erhalten werden können oder aber gezielt gasförmige Kohlenwasserstoffe generiert werden können.

Beispielsweise offenbaren die DE 103 27 059 A1 und die EP 1 724 325 A1 Verfahren, bei denen fetthaltige Ausgangsmaterialien bei erhöhten Temperaturen in einem Reaktor mit einem Aktivkohle-Festbett in Kontakt gebracht werden. Hierbei erfolgt eine Decarboxylierung der enthaltenen Fettsäuren, so dass flüssige Kohlenwasserstoffe entstehen. Um zu gasförmigen Kohlenwasserstoffen zu gelangen, können die Verfahrensparameter und gegebenenfalls auch die Aufbereitung des Ausgangsmaterials modifiziert werden, so dass ein Abbau der Ausgangsmaterialien zu gasförmigen, kurzkettigen Kohlenwasserstoffen erfolgt. Eine verstärkte Bildung von Kohlenwasserstoffen mit mittleren Kettenlängen ist in den genannten Schriften aber nicht beschrieben.

Mit dem Fischer-Tropsch-Verfahren lassen sich zwar Kohlenwasserstoffe mit beliebiger Kettenlänge aus Fetthaltigen Ausgangsmaterialien herstellen; allerdings wird bei diesem Verfahren das Ausgangsmaterial zunächst zu $C_1$-Bruchstücken abgebaut, aus denen nachfolgend Kohlenwasserstoffe mit unterschiedlicher Kettenlänge erst wieder aufgebaut werden müssen. Dieses Verfahren ist demnach mit einem erheblichen Energieaufwand verbunden und zudem nur in sehr großem Maßstab wirtschaftlich sinnvoll.

J. Fu et al. (ACS Catalysis 2011, 1 (3), S. 227-231) beschreibt die Decarboxylierung von Ölsäure und Palmitin zu Kohlenwasserstoffen, die für Kraftstoffe geeignet sind. Das dort beschriebene Verfahren erfolgt in Gegenwart von Aktivkohle-Katalysatoren bei erhöhten Temperaturen.

Es ist eine Aufgabe der vorliegenden Erfindung, die Nachteile des Stands der Technik zu überwinden und ein Verfahren anzugeben, mit dem sich sauerstoffhaltige Kohlenwasserstoffverbindungen oder Gemische von sauerstoffhaltigen Kohlenwasserstoffverbindungen in Kohlenwasserstoffe mit mittleren Kettenlängen umwandeln lassen. Eine weitere Aufgabe der vorliegenden Erfindung ist es, ein Verfahren zur Herstellung von Kohlenwasserstoffgemischen bereitzustellen, das zur Herstellung von Bio-Kerosin geeignet ist.

Zumindest eine dieser Aufgaben wird durch den Gegenstand der unabhängigen Ansprüche gelöst. Weitere Ausgestaltungen und Weiterbildungen sind Gegenstand von Unteransprüchen und gehen weiterhin auch aus der nachfolgenden Beschreibung hervor.

Ein Verfahren zur Gewinnung von reinen Kohlenwasserstoffen mit mittleren Kettenlängen oder Kohlenwasserstoffgemischen mit einem erhöhten Anteil von Kohlenwasserstoffen mit mittleren Kettenlängen umfasst folgende Schritte:

A) Zunächst wird ein Ausgangsmaterial bereitgestellt, das mindestens 50 Gew.-% ungesättigte sauerstoffhaltige Kohlenwasserstoffverbindungen enthält. Diese ungesättigten Kohlenwasserstoffverbindungen weisen zumindest zum Teil Struktureinheiten auf, die der nachfolgenden Spezifikation genügen (diese Struktureinheiten werden - da sie zumindest teilweise für den ungesättigten Charakter der Kohlenwasserstoffverbindungen verantwortlich sind - nachfolgend Olefinfragmente genannt). Die Olefinfragmente sind einfach ungesättigt und besitzen die Formel $-C_1-C_xH_{2x}-CH=CH-C_yH_{2y+1}$.(Formel I) Das Kohlenstoffatom $C_1$ in diesem Olefinfragment markiert den Übergang zu einem Heteroatom. Dementsprechend ist das Kohlenstoffatom $C_1$ mit zumindest einem substituierten oder unsubstituierten Heteroatom und gegebenenfalls auch Wasserstoff abgesättigt. Häufig wird an das Kohlenstoffatom $C_1$ ein Sauerstoffatom gebunden sein, das seinerseits dann wiederum an ein Kohlenstoffatom (wie beispielsweise in einem Fettsäureester, etwa einem Glycerid) gebunden ist oder zusammen mit dem Kohlenstoffatom $C_1$ Bestandteil einer Carboxylatgruppe ist (wie z.B. in einer Fettsäure). Die Indizes x und y in der genannten Formel sind ganze Zahlen, wobei x größer ist als 1 und y größer ist als 0. Mit anderen Worten handelt es sich weder um ein Olefinfragment mit endständiger Doppelbindung noch um ein Olefinfragment, bei dem die Doppelbindung direkt auf das $C_1$-Atom folgt. Die Olefin-fragmente sind ferner dadurch gekennzeichnet, dass sie zumindest 14 Kohlenstoffatome aufweisen, insbesondere 16 bis 22 Kohlenstoffatome.

Insbesondere bei Ausgangsmaterialien, die die Gemische verschiedener ungesättigter sauerstoffhaltiger Kohlenwasserstoffverbindungen enthalten, aber gegebenenfalls auch bei Ausgangsmaterialien, die nur genau eine sauerstoffhaltige Kohlenwasserstoffverbindung enthalten, welche ein Di- oder Triglycerid ist, werden mehrere unterschiedliche Olefinfragmente vorliegen. Nachfolgend wird dabei das Olefinfragment, mit dem größten molaren Anteil mit $k_1$ mol-% als Hauptkomponente $K_1$ bezeichnet. Weitere Olefinfragmente werden mit $K_2$ bis $K_x$ bezeichnet und besitzen einen Anteil von $k_2$ bis $k_x$ mol-%. Für die Bestimmung des erfindungsgemäß erzielten erhöhten Anteils von Kohlenwasserstoffen mittlerer Kettenlänge werden hierbei nur solche Olefinfragmente erfasst, die zumindest in einem Anteil von 5 mol-% vorliegen. Mittels dieser Maßgabe wird erreicht, dass eine einfache Bestimmung eines erhöhten Anteils möglich ist, zumal Olefinfragmente mit geringerem Anteil nur noch eine geringe Auswirkung auf das Produktspektrum ausüben.

Neben dem Ausgangsmaterial wird auch ein poröser Katalysators auf Kohlenstoff-Basis mit einem Mikroporen-Anteil, der mit zumindest 800 m$^2$/g zur BET-Oberfläche beiträgt, bereitgestellt;

Im Schritt B) des erfindungsgemäßen Verfahrens wird das Ausgangsmaterial in Abwesenheit von Sauerstoff bei einem Druck von 20 bis 20000 hPa und bei erhöhter Temperatur in einem Konvertierungsmaterial mit dem porösen Katalysator kontaktiert. Als erhöhte Temperatur ist hierbei insbesondere eine Temperatur von 200-800°C anzusehen, bevorzugt 300-600°C und insbesondere 350-550°C. Unter derartigen Bedingungen erhält man mit dem gewählten Ausgangsmaterial ein Produktgemisch, in dem der Anteil an Kohlenwasser

stoffen mit mittleren Kettenlängen, d.h. insbesondere der Anteil zumindest eines (gegebenenfalls reinen) Kohlenwasserstoffs mit 8 bis 16 Kohlenstoffatomen einen erhöhten Anteil aufweist.

In einem Teilschritt C) wird schließlich das kohlenwasserstoffhaltige Produktgemisch aufgefangen und einer Separiereinrichtung zugeführt, in der eine Produktauftrennung erfolgt.

Unter einem erhöhten Anteil von Kohlenwasserstoffen mit mittleren Kettenlängen wird erfindungsgemäß verstanden, dass im Produktspektrum zumindest der Anteil einer der Komponenten mit 8, 9, 10, 11, 12, 13, 14, 15 oder 16 oder mehr Kohlenstoffatomen erhöht ist. Meist ist der Anteil von zumindest zwei dieser Komponenten erhöht, häufig auch von drei oder mehr als drei dieser Komponenten. Insbesondere tritt die Komponente mit y+3 Kohlenstoffatomen im Produktspektrum verstärkt auf (wobei sich y auf die vorstehend angegebene Formel des Olefinfragments bezieht). Für die Hauptkomponente $K_1$ und die weiteren Komponenten $K_2$ - $K_x$ wird dazu jeweils die zugehörige Komponente mit y+3 Kohlenstoffatomen ermittelt, die dementsprechend gewissermaßen als "Marker" dient. Ein erhöhter Anteil kann erfindungsgemäß auf zwei unterschiedliche Arten detektiert werden, wobei der nachfolgend erläuterten ersten Alternative aufgrund der einfacheren Zugänglichkeit der nötigen Daten der Vorzug zu geben ist.

Gemäß der ersten Alternative wird ein erhöhter Anteil von Kohlenwasserstoffen mit mittleren Kettenlängen folgendermaßen bestimmt: zunächst werden für die Hauptkomponente $K_1$ und jede weitere gegebenenfalls heranzuziehende Komponente $K_2$ bis $K_x$ (mit mehr als 5 mol-% Anteil) die aliphatischen Produktverbindungen $\beta$, die alle y+3 Kohlenstoffatome aufweisen, ermittelt (für die Hauptkomponente also die Produktverbindungen $\beta_1$, für die weitere Komponente $K_2$ die Produktverbindungen $\beta_2$ usw.). Hierbei handelt es sich um die Markerverbindungen, welche einen besonders hohen Anteil aufweisen, wobei als aliphatische Produktverbindungen $\beta$ neben den unverzweigten gesättigten Verbindungen nur die einfach ungesättigten Produktverbindungen des Produktgemischs erfasst werden, und auch nur dann, wenn ihr Gewichtsanteil im Produktgemisch mehr als 10 %, bezogen auf die gesättigte unverzweigte Produktverbindung, beträgt (niedrigere Gehalte verkomplizieren das Bestimmungsverfahren für den erhöhten Anteil unnötig und tragen auch zum Ergebnis dieses Bestimmungsverfahrens nicht signifikant bei, werden sinnvollerweise also weggelassen). Die aliphatischen Produktverbindungen $\beta_x$ mit y+3 Kohlenstoffatomen entsprechen also einem Kohlenwasserstoff, der sich formal durch Brechen der Bindung in $\beta$-Position zur Doppelbindung auf der vom Kettenende abgewandten Seite ergibt (wobei auch die Produktverbindungen miteinbezogen sind, bei denen formal auch noch eine Hydrierung oder Isomerisierung der Doppelbindung erfolgen muss). Erfindungsgemäß wurde festgestellt, dass Verbindungen mit einer derartigen Kettenlänge besonders verstärkt gebildet werden, wenn das vorstehend genannte Ausgangsmaterial Verwendung findet. Die formale Bildung durch Brechen der Bindung in $\beta$-Position soll hierbei allerdings nicht einschränkend verstanden werden, sondern wird lediglich für die Definition der Produktverbindungen $\beta_x$ herangezogen.

Weiterhin werden für die Hauptkomponente $K_1$ und jede weitere gegebenenfalls heranzuziehende Komponente $K_2$-$K_x$ auch die korrespondierenden Produktverbindungen $\delta$, die alle y-1 Kohlenstoffatome aufweisen, ermittelt (für die Hauptkomponente also die Produktverbindungen $\delta_1$, für gegebenenfalls vorhandene weitere Komponenten die Produktverbindungen $\delta_2$ usw.). Auch hierbei werden wie vorstehend für die Produktverbindungen $\beta$ die unverzweigten gesättigten aliphatischen Produktverbindungen ermittelt sowie die unverzweigten einfach ungesättigten aliphatischen Produktverbindungen, sofern deren molarer Anteil mehr als 10 %, bezogen auf die gesättigten aliphatischen Produktverbindungen, beträgt. Die Produktverbindungen $\delta$ stehen für eine Produktkomponente, die in weniger verstärktem Ausmaß oder nicht verstärkt gebildet werden und stellen somit einen "Antimarker" dar. Sie entsprechen dem Bruchstück, das gebildet wird, wenn in $\alpha$-Position zur Doppelbindung auf der Seite des Kettenendes des Produktfragments formal eine Bindungstrennung erfolgt. Erfindungsgemäß wurde beobachtet, dass diese Produktverbindungen $\delta$ bei dem oben beschriebenen Ausgangsmaterial in besonders geringem Ausmaß (bezogen auf die Produktverbindungen $\beta$) gebildet werden.

Gemäß der ersten Alternative zur Bestimmung des erhöhten Anteils werden dann in einem Teilschritt b) für alle so ermittelten Produktverbindungen $\beta_x$ und $\delta_x$ ihre im Produktgemisch vorliegenden molaren Anteile $n_{\beta x}$ und $n_{\delta x}$ ermittelt. Bei einem Ausgangsmaterial, in dem eine Hauptkomponente und zwei weitere Komponenten vorliegen, werden also für die Produktverbindungen $\beta_1$, $\beta_2$ und $\beta_3$ ihre Anteile $n_{\beta 1}$, $n_{\beta 2}$ und $n_{\beta 3}$ im Produktgemisch ermittelt und für $\delta_1$, $\delta_2$ und $\delta_3$ ihre Anteile $n_{\delta 1}$, $n_{\delta 2}$ und $n_{\delta 3}$.

Gemäß der ersten Alternative wird in einem Teilschritt c) schließlich aus allen Produktverbindungen $\beta_x$ der gemittelte Anteil $\overline{n_\beta}$ und für die Produktverbindungen $\delta_x$ der gemittelter Anteil $\overline{n_\delta}$ gerechnet, wobei der gemittelte Anteil gemäß der Formel für die Berechnung des Zahlenmittels der Molmasse eines Polymers Verwendung findet, nämlich

$$\overline{n} = \sum_{i=1}^{x} k_i * n_i$$ . Liegen eine Hauptkomponente und zwei weitere Komponenten vor, wird also für die drei Markerproduktverbindungen $\beta$ der gemittelte Anteil $\overline{n_\beta}$ abhängig vom jeweils bestimmten molaren Anteil der Hauptkomponente und der weiteren Komponenten im Ausgangsmaterial und ebenso für den Antimarker, d.h. die Produktverbindungen $\delta$, der gemittelte Anteil $\overline{n_\delta}$ aus den drei Antimarkerproduktverbindungen.

Ein erhöhter Anteil von Kohlenwasserstoffen mit mittleren Kettenlängen (also Kohlenwasserstoffen mit 8 bis 16 Kohlenstoffatomen) liegt erfindungsgemäß dann vor, wenn gilt, dass $\overline{n_\beta} > 1{,}15 * \overline{n_\delta}$, d.h. dass der mittlere Anteil des Markers zumindest 15 % höher ist als der mittlere Anteil des Antimarkers. Bevorzugt gilt, dass $\overline{n_\beta} > 1{,}25 * \overline{n_\delta}$ und besonders bevorzugt, dass $\overline{n_\beta} > 1{,}5 * \overline{n_\delta}$; der mittlere Anteil des Markers ist also um mindestens 25% bzw. 50 % höher als der des Antimarkers.

Gemäß einer zweiten Alternative kann ein erhöhter Anteil von Kohlenwasserstoffen mit mittlerer Kettenlänge auch folgendermaßen detektiert werden: Wie vorstehend in der ersten Alternative werden für ein gegebenes Ausgangs-material die gemittelten Anteile $\overline{n_\beta}$ für die Produktverbindungen $\beta_x$ der Hauptkomponente und aller gegebenenfalls vorhandenen Nebenkomponenten ermittelt. Anschließend wird das Ausgangsmaterial mittels Wasserstoff/Palladium-Katalysator so hydriert, dass der Anteil der Doppelbindungen im Ausgangsmaterial um mindestens 95% vermindert wird (Ermittlung anhand der Iodzahl). Dieses Material wird analog zum erfindungsgemäßen Verfahren unter denselben Reaktionsbedingungen wie das nicht hydrierte Ausgangsmaterial zu einem Produktgemisch umgesetzt, in welchen dann wieder für dieselben Produktverbindungen $\beta$ wie sie für das nicht hydrierte Ausgangsmaterial ermittelt wurden) der gemittelte Anteil $\overline{n_\beta}\#$ ermittelt. Es gilt dann, dass $\overline{n_\beta} > 1{,}15 * \overline{n_\beta}\#$ (beim nicht hydrierten Ausgangsmaterial wird mindestens 15 % des Markers mehr gebildet). Bevorzugt gilt, dass $\overline{n_\beta} > 1{,}25 * \overline{n_\beta}\#$ und besonders bevorzugt, dass $\overline{n_\beta} > 1{,}5 * \overline{n_\beta}\#$ (also zumindest 25% bzw. 50 % mehr des Markers gebildet wird).

Das erfindungsgemäße Verfahren bietet erstmalig die Möglichkeit, bei der Spaltung von Verbindungen wie Fetten und Ölen an einem porösen kohlenstoffhaltigen Katalysator durch gezielte Wahl des Ausgangsmaterials hohe Ausbeuten an Kohlenwasserstoffen mit mittleren Kettenlängen, also Kohlenwasserstoffen von 8 bis 16 Kohlenstoffatomen zu erhalten. Die Verfahren nach dem Stand der Technik sind im Wesentlichen darauf ausgelegt, Fette und ähnliche Stoffe zu langkettigen Kohlenwasserstoffen zu spalten bzw. führen zu Produkten, bei denen im verstärkten Ausmaß lediglich eine Abspaltung der Carboxylatgruppe von Fettsäuren beobachtet wird. Die Verfahren nach dem Stand der Technik fokussieren also insbesondere darauf, aus einem Ausgangsmaterial reine Kohlenwasserstoff-Verbindungen zu erhalten, indem aus den Ausgangsmaterialien die Heteroatome abgespalten werden. Eine Spaltung von Kohlenstoff-Kohlenstoff-Bindungen findet häufig nur im beschränkten Umfang statt oder ist sogar unerwünscht, wenn man davon absieht, dass die Reaktionsbedingungen auch so drastisch sein können, dass verstärkt nur Bruchstücke mit 1 bis 4 Kohlenstoffatomen gebildet werden. Es wird dagegen nicht Beschrieben, dass nach Abspaltung beispielsweise der Carboxylatgruppe einer Fettsäure in verstärktem Umfang (nur) ein weiterer Bindungsbruch erfolgt und dementsprechend eine verstärkte Bildung von Kohlenwasserstoffen mit mittleren Kettenlängen.

Beim erfindungsgemäßen Verfahren wurde ferner erkannt, dass durch gezielten Einsatz von Doppelbindungs-haltigen Ausgangsmaterialien ein Produktspektrum erhalten wird, in dem sich die Kohlenwasserstoffe in verstärktem Maße nachweisen lassen, welche sich durch den Bruch von Bindungen ergeben, die benachbart zu Doppelbindungen sind. Mit anderen Worten wurde erfindungsgemäß erkannt, dass durch den Einsatz von Doppelbindungs-haltigen Ausgangsmaterialien kürzere Produkte erhalten werden als beim Einsatz von den korrespondierenden Verbindungen, die statt einer Doppelbindung eine Einfachbindung aufweisen d.h. gesättigt sind.

Erfindungsgemäß hat sich schließlich herausgestellt, dass insbesondere einfach ungesättigte Olefinfragmente besonders geeignet sind, um das gewünschte Produktspektrum zu erreichen. Bei mehrfach ungesättigten Verbindungen ist häufig zu beobachten, dass bei den in Teilschritt B) herrschenden Bedingungen eine Polymerisation oder andere chemische Reaktionen der Doppelbindungen stattfinden, so dass beim Cracken von mehrfach ungesättigten Olefinfragmenten im Regelfall keine verstärkte Bildung von Kohlenwasserstoffen mit mittleren Kettenlängen zu beobachten ist.

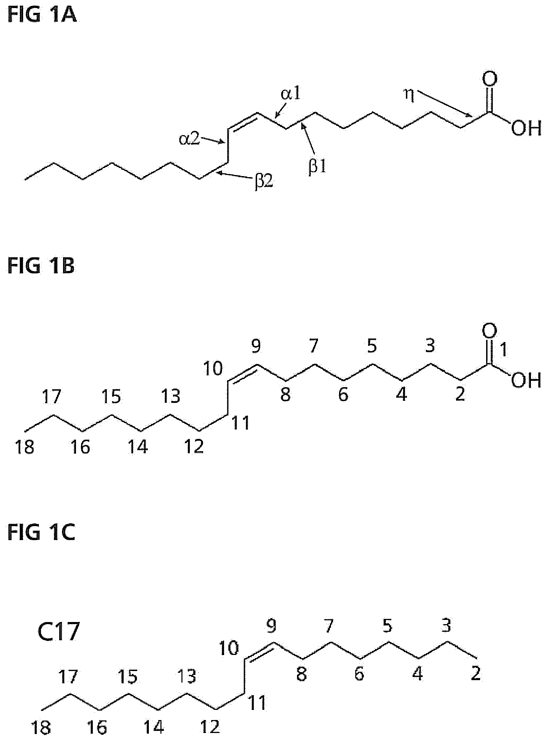

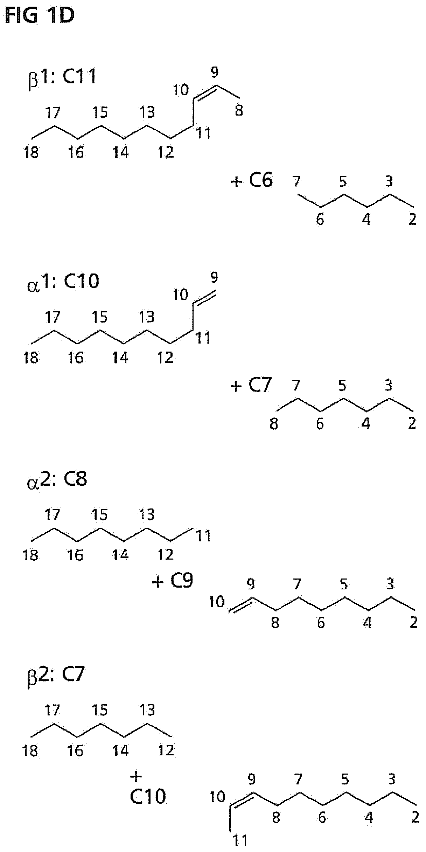

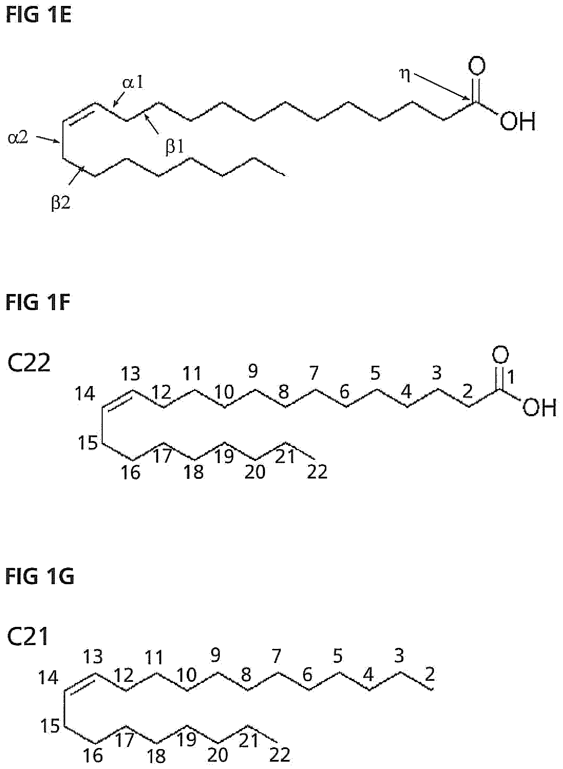

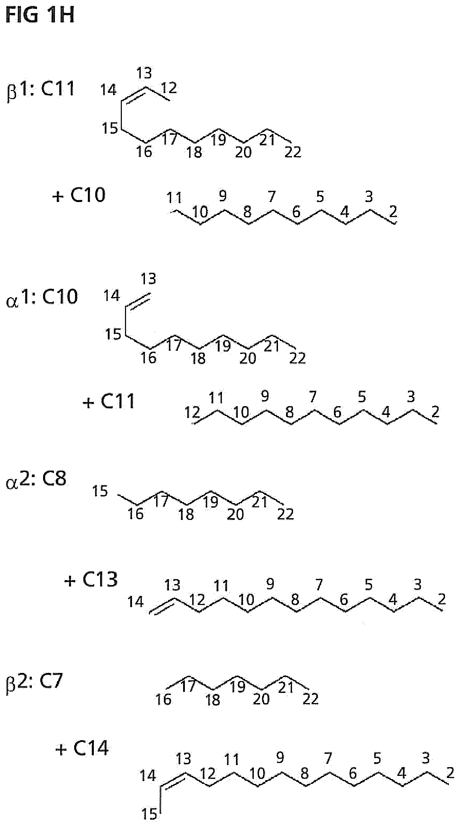

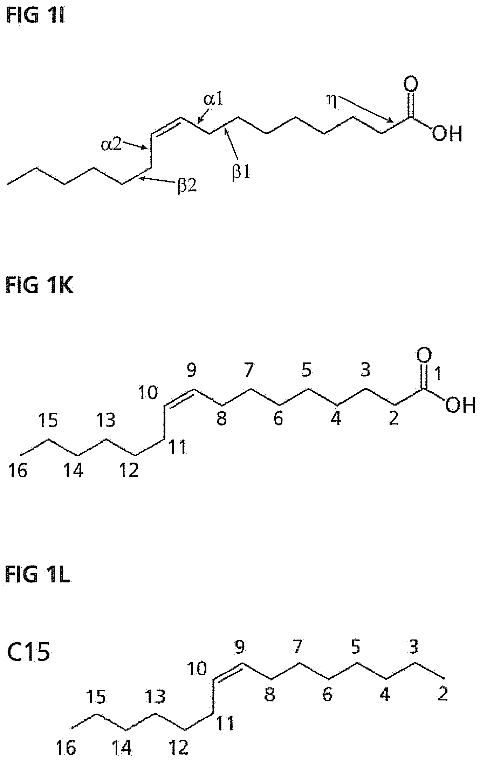

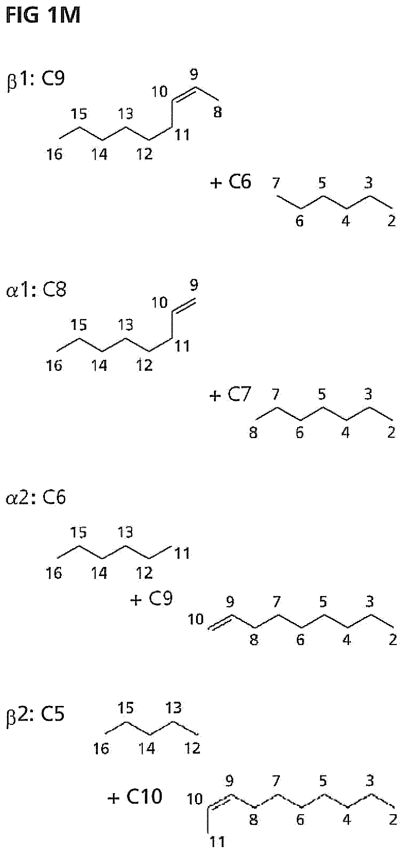

Das erhaltene erfindungsgemäß erhaltene Produktspektrum kann besonders gut anhand der Bruchstücke charakterisiert werden, welche formal durch Bruch der Bindung erhalten werden, die in $\beta 1$-Position zur Doppelbindung steht. Im Gegensatz dazu ist ein Bruch der Bindung in $\alpha 2$-Position (vgl. Figur 1) wesentlich weniger signifikant, so dass sich der (zum Kettenende hin) verbleibende Kohlenwasserstoffrest als Antimarker eignet. Da im Ausgangsmaterial häufig ein Gemisch vorliegt, ist erfindungsgemäß ein mathematisch aufwändiges Verfahren durchzuführen, um den Anteil dieser Bruchstücke bestimmen zu können.

Zur Erläuterung sei auf die Figuren 1A bis 1M verwiesen. Figur 1A zeigt Ölsäure als Ausgangsmaterial und die Bindungen in $\alpha$1-, $\alpha$2-, $\beta$1- und $\beta$2-Position zur Doppelbindung. Figur 1B zeigt dasselbe Molekül mit Durchnummerierung der Kohlenstoffkette. Beim katalytischen Cracken nach dem Stand der Technik würde man in verstärktem Ausmaß lediglich ein Brechen der Verbindung in $\eta$-Position (vgl. Figur 1A) erwarten, so dass sich ein C17-Fragment ergibt (vgl. Figur 1C). Beim erfindungsgemäßen Verfahren wird dagegen ein anderes Produktspektrum beobachtet; hier wird neben einem Produkt mit 11 Kohlenstoffatomen, das sich formal durch Spaltung der $\beta$1-Bindung ergibt auch eine verstärkte Bildung eines Produkts mit 10 Kohlenstoffatomen beobachtet, das sich formal durch Spaltung der $\alpha$1-Bindung ergibt. Figur 1D zeigt die zu erwartenden Produkte, die sich bei Spaltung der $\alpha$1-, $\alpha$2-, $\beta$1- und $\beta$2-Bindung in Figur 1A ergeben. Von den jeweils zwei erwarteten Produkten ist hierbei insbesondere die Bildung des jeweils längerkettigen Produkts zu beobachten, in Figur 1D dementsprechend die verstärkte Bildung von C10- und C11- Produkten. Die Figuren 1E bis 1H zeigen die entsprechenden Ergebnisse bei Erucasäure als Ausgangsmaterial (Figur 1 E) mit der Nummerierung gemäß Figur 1 F und dem nach dem Stand der Technik zu erwartenden Hauptprodukt beim katalytischen Cracken, der C21-Fraktion (Figur 1G) sowie den zu erwartenden Produkten bei $\beta$1-, $\alpha$1-, $\alpha$2- und $\beta$2-Spaltung (Figur 1 H). Erfindungsgemäß bildet sich insbesondere C11- und C10 ($\beta$1-Spaltung und $\alpha$1-Spaltung). Figur 1I bis 1M zeigt schließlich noch das Beispiel Palmitoleinsäure (Figur 1I mit Nummerierung Figur 1 K), nach dem Stand der Technik zu erwartendes Primärprodukt beim Cracken (Figur 1 L) und den Spaltprodukten, die sich durch Spalten der $\alpha$1-, $\alpha$2-, $\beta$1- und $\beta$2-Bindung ergeben (Figur 1 M). Bei verstärkter $\beta$1-und $\alpha$1-Spaltung werden hierbei die Fragmente C8 und C9 verstärkt gebildet.

Als Ausgangsmaterialien werden erfindungsgemäß sauerstoffhaltige Kohlenwasserstoffe oder Kohlenwasserstoffgemische eingesetzt. Als Ausgangsstoffe sind insbesondere biogene Ausgangsstoffe zu nennen. Zu erwähnen sind etwa Lipide und fettartige Verbindungen, wobei unter Ausgangsstoffen, die Lipide und/oder fettartige Verbindungen enthalten, verstanden wird, dass die Ausgangsstoffe Lipide und/oder wesentliche Bestandteile von Lipiden (wie Mono- und Diglyceride) enthalten oder hieraus bestehen. Die Ausgangsmaterialien enthalten dabei erfindungsgemäß mindestens 50 Gew.-% ungesättigte sauerstoffhaltige Kohlenwasserstoff-Verbindungen, wobei dieser Anteil beispielsweise mittels Gaschromatographie ermittelt werden kann. Diese ungesättigten sauerstoffhaltigen Kohlenwasserstoffverbindungen enthalten die vorstehend definierten Olefinfragmente. Um nun den Anteil der Hauptkomponente und den Anteil der gegebenenfalls relevanten weiteren Komponenten $K_2$ bis $K_x$ zu bestimmen, wird die aus der Biodieselherstellung bekannte Fettsäure Methylester-Bestimmung (" FAME") gemäß dem Standard EN14214 durchgeführt. Hierbei können dann auch für Ausgangsmaterialien, die neben Fettsäuren und Fettsäureestern auch andere ungesättigte sauerstoffhaltige Kohlenwasserstoffverbindungen enthalten, die Gehalte der erfindungsgemäßen Olefinfragmente ermittelt werden.

Die ungesättigten sauerstoffhaltigen Kohlenwasserstoff-Verbindungen können unverzweigte oder verzweigte Verbindungen sein. Häufig liegt ein hoher Anteil verzweigter Verbindungen vor, da auch der Anteil der Triglyceride häufig hoch sein wird. Darüber hinaus können auch verschiedene in den einzelnen Ausgangsmaterialien enthaltene Olefinfragmente entweder vollständig unverzweigt sein oder es können neben unverzweigten auch verzweigte vorliegen (im Einzelfall ist auch denkbar nur verzweigte Olefinfragmente einzusetzen). Eine derartige Verzweigung der Olefinfragmente kann insbesondere darin bestehen, dass genau eine Verzweigung vorliegt. Beispielsweise kann eine Verzweigung in Form von Methyl-Gruppen im Olefinfragment vorliegen, wobei diese Verzweigung entweder im Teilfragment $C_xH_{2x}$ oder $C_yH_{2y+1}$ vorliegt.

Gemäß einer Ausführungsform werden die Olefinfragmente der ungesättigten sauerstoffhaltigen Kohlenwasserstoffverbindungen, die als Ausgangsmaterial eingesetzt werden, so ausgewählt, dass die Olefinfragmente im Wesentlichen einen Bestandteil $C_{y'}H_{2y+1}$ enthalten, bei dem y zumindest 5, insbesondere zumindest 6 und besonders bevorzugt zumindest 7 ist. Im Wesentlichen heißt hierbei, dass zumindest 90 mol-% der Olefinfragmente in dieser Form vorliegen. Mit Ausgangsmaterialien, bei den y zumindest 5, zumindest 6 oder zumindest 7 ist, werden mittels der $\beta$1-Spaltung Kohlenwasserstofffragmente gebildet, bei denen die Kettenlänge 8, 9 oder 10 Kohlenstoffatome beträgt. Es werden also - zumindest mit der $\beta$1-Spaltung - Kohlenwasserstoffe mit mittleren Kettenlängen gebildet.

Um zu einem hohen Anteil von Kohlenwasserstoffen mit mittleren Kettenlängen zu gelangen, sollte der Anteil der ungesättigten sauerstoffhaltigen Kohlenwasserstoffverbindungen im Ausgangsmaterial besonders hoch sein. Da davon auszugehen ist, dass häufig Ausgangsmaterialien, die einen großen Anteil Fette und Öle enthalten, eingesetzt werden, beispielsweise einen Anteil von >75 Gew.-% Fetten und Ölen, beispielsweise >90 Gew.-% Fetten und Ölen, kann auch die mittels der Methylesterbestimmung (die wie vorstehend erläutert bei der Biodieselherstellung Verwendung findet) dazu dienen, Ausgangsmaterialien zu charakterisieren, die sich besonders gut für die Gewinnung von Kohlenwasserstoffen mit mittleren Kettenlängen eignen. Gemäß einer Ausführungsform ist daher der im Ausgangsmaterial enthaltenen einfach ungesättigten Fettsäuren mit mindestens 14 Kohlenstoffatomen an den gesamten im Ausgangsmaterial enthaltenen Fettsäuren zumindest 30 mol-%, insbesondere zumindest 50 mol-%, beispielsweise zumindest 75 mol-%.

Unter einem porösen Katalysator auf Kohlenstoff-Basis wird erfindungsgemäß ein Stoff verstanden, dessen Oberfläche für das zu konvertierende Ausgangsmaterial bzw. die darin enthaltenen Verbindungen zugängliche Poren besitzt und der in der Lage ist, das Ausgangsmaterial bzw. die darin enthaltenen Verbindungen zu spalten bzw. die Spaltung katalytisch zu unterstützen. Als Katalysatoren sind hierbei insbesondere feinporige kohlenstoffhaltige Materialien zu nennen (also Substanzen mit Poren mit einem Durchmesser kleiner oder gleich 20 $\mu$m). Hiervon sind erfindungsgemäß Stoffe, die Mesoporen und/oder Mikroporen und/oder Submikroporen enthalten, bevorzugt (gemäß der IUPAC-Definition haben Mesoporen einen Porendurchmesser von 2-50 nm, Mikroporen einen Porendurchmesser von 0,4-2 nm und Submikroporen einen Porendurchmesser <0,4 nm). Ferner kommen als poröse Katalysatoren insbesondere im Wesentlichen aus Kohlenstoff bestehende Katalysatoren und andere Katalysatoren auf Kohlenstoff-Basis in Betracht. Unter "auf Kohlenstoff-Basis" wird erfindungsgemäß verstanden, dass (in Bezug auf Oberfläche und Reaktionsdauer) zumindest 90% der während der Durchführung von Schritt B) katalytisch aktiven Oberfläche aus Kohlenstoff und/oder aus polycyclischen aromatischen Kohlenwasserstoffen besteht. Häufig wird zumindest 99% der katalytisch aktiven Oberfläche und oft auch die gesamte katalytisch aktive Oberfläche aus Kohlenstoff bestehen. Dieses Kriterium ist bei im Wesentlichen aus Kohlenstoff bestehenden Katalysatoren sowie bei Katalysatoren, die im Wesentlichen aus anorganischen Stoffen bestehen, im Regelfall dann erfüllt, wenn die Dauer von Schritt B) (und damit die Einsatzzeit des Katalysators) zumindest 30 Minuten beträgt. Unter "Wesentlichen bestehend aus" wird ein Katalysator verstanden, der mindestens 90 Gew.-%, insbesondere mindestens 95 Gew.-% anorganische Stoffe bzw. Kohlenstoff aufweist.

Unter einem Kontaktieren des Ausgangsmaterials in Abwesenheit von Sauerstoff kann erfindungsgemäß auch verstanden werden, dass neben den Ausgangsmaterialien und gegebenenfalls Inertgas im Regelfall keine weiteren Gase dem Reaktor zugeführt werden. Insbesondere wird dem Reaktor im Regelfall kein Hydrierungsmittel, wie es beispielsweise beim Hydrocracking eingesetzt wird, zugeführt. Davon unabhängig kann während des Teilschritts B) aber durch die Reaktion des Ausgangsmaterials mit dem Katalysator ein Produktspektrum entstehen, in dem Verbindungen vorliegen, die selbst wiederum einen reduzierenden Charakter besitzen (beispielsweise Kohlenmonoxid). Gemäß einer Ausführungsform der Erfindung weisen die in den ungesättigten sauerstoffhaltigen Kohlenwasserstoff-Verbindungen vorliegenden Olefinfragmente (gemäß Formel I) mit mindestens 14 Kohlenstoffatomen einen Anteil >75 mol-% an Komponenten K auf, für die gilt, dass x > 4 ist. Der Anteil der Komponenten K wird hierbei wie vorstehend erläutert bestimmt. Gemäß dieser Ausführungsform liegt also im Ausgangsmaterial ein erhöhter Anteil der Doppelbindungen in Kohlenwasserstoffketten vor, bei denen zwischen dem an das $C_1$-Atom gebundenen Heteroatom und der Doppelbindung mindestens 6 Kohlenstoffatome vorliegen. In diesen Verbindungen kann dann ein zusätzlicher ein "Anti-Marker" definiert werden. Es handelt sich hierbei um die aliphatischen Produktverbindungen $\varepsilon_x$ die y+6 Kohlenstoffatome besitzen. Auch hier gilt wie vorstehend, dass als aliphatische Produktverbindungen $\varepsilon_x$ zunächst die unverzweigten gesättigten Produktverbindungen erfasst werden und ferner alle einfach ungesättigten aliphatischen Produktverbindungen mit y+6 Kohlenstoffatomen, deren molarer Anteil zumindest 10 % der Menge der gesättigten Produktverbindung mit y+6 Kohlenstoffatomen beträgt. Diese Produktverbindungen $\varepsilon_x$ können allerdings nur bei Ausgangsmaterialien mit x > 4 als "Anti-Marker" dienen, da bei kleinerem x eine Störung durch Spaltprodukte der ausschließlichen Spaltung in der $\eta$-Position (vgl. Figur 1A, 1E und 1I) erfolgt, also durch das Produkt, welches durch die reine Decarboxylierung gebildet wird, bei dem noch keine Spaltung einer Bindung in der Nähe der Doppelbindung erfolgt ist. Die Bestimmung der Produktverbindungen $\varepsilon_x$ erfolgt dabei gemäß der vorstehend erläuterten ersten Alternative zur Bestimmung eines erhöhten Anteils von Kohlenwasserstoffen mit mittleren Kettenlängen.

Gemäß dieser ersten Alternative kann dann gegebenenfalls auch der zweite "Anti-Marker" zur Charakterisierung eines erhöhten Anteils der mittleren Kettenlängen dienen. Auch für die Produktverbindungen $\varepsilon_x$ kann dann wie vorstehend (gemäß der ersten Alternative) der Anteil $n_{\varepsilon x}$ der Hauptkomponente und der gegebenenfalls vorhandenen weiteren Komponenten $K_2$ bis $K_x$ bestimmt werden und auch der gemittelte Anteil $\overline{n_\varepsilon}$ ermittelt werden. Werden die Produktverbindungen $\varepsilon_x$ als Anti-Marker herangezogen, so gilt dann zusätzlich, dass $\overline{n\beta} > \overline{n_\varepsilon}$ (der mittlere Anteil der Marker-Komponente ist also größer als der mittlere Anteil der zweiten Anti-Marker-Komponente). In den allermeisten Fällen gilt zudem, dass

$\overline{n_\beta} > 1,15 * \overline{n_\varepsilon}$ (der Anteil des Markers ist also um mindestens 15 % höher als der des zweiten Anti-Markers) und in den meisten Fällen gilt ferner, dass $\overline{n_\beta} > 1,25 * \overline{n_\varepsilon}$ und häufig auch, dass $\overline{n_\beta} > 1,5 * \overline{n_\varepsilon}$ (der Anteil des Markers ist also um mindestens 25% bzw. 50 % höher als der des Anti-Markers).

Gemäß einer weiteren Ausführungsform kann auch noch ein zweiter Marker verwendet werden. Erfindungsgemäß hat sich gezeigt, dass nicht nur die Produktverbindungen mit y+3 Kohlenstoffatomen in verstärktem Maß gebildet werden, sondern meist auch noch die Produktverbindungen mit y+2 Kohlenstoffatomen (wenn auch häufig der mittlere Anteil der Produktverbindungen mit y+2 Kohlenstoffatomen kleiner ist als der der Produktverbindungen mit y+3 Kohlenstoffatomen). Wie vorstehend für den Marker in der ersten Alternative zur Bestimmung des erhöhten Anteils und Kohlenwasserstoffen mit mittleren Kettenlängen beschrieben, können auch für den zweiten Marker, die unverzweigten und gesättigten oder einfach ungesättigten aliphatischen Produktverbindungen $\gamma_x$ mit y+2 Kohlenstoffatomen (die wiederum wie vorstehend definiert bestimmt werden bzw. hinsichtlich der ungesättigten Produktverbindungen erfasst oder nicht erfasst werden) der Anteil $n_{\gamma x}$ für die Hauptkomponente $K_1$ und gegebenenfalls vorliegenden weiteren Komponenten

$K_2$ bis $K_x$ jeweils bestimmt werden und hieraus der gemittelte Anteil $\overline{n_\gamma}$ ermittelt werden. Wird auch noch der zweite Marker herangezogen, so gilt zusätzlich dass $\overline{n_\gamma} > \overline{n_\delta}$ und (sofern der Anteil der Komponenten K mit x > 4 an den Olefinfragmenten > 75 mol-% ist) auch $\overline{n_\gamma} > \overline{n_\varepsilon}$. Der Anteil des zweiten Markers ist also größer als der des Anti-Markers und des zweiten Anti-Markers. In den meisten Fällen gilt ferner, dass $\overline{n_\gamma} > 1{,}15 * \overline{n_\delta}$, insbesondere $\overline{n_\gamma} > 1{,}25 * \overline{n_\delta}$, und unabhängig hiervon - sofern die Komponenten gegebenenfalls $\varepsilon_x$ erfasst werden - auch $\overline{n_\gamma} > 1{,}15 * \overline{n_\varepsilon}$, insbesondere $\overline{n_\gamma} > 1{,}25 * \overline{n_\varepsilon}$. (Der Anteil des Markers ist also in den meisten Fällen um 15% bzw. 25 % größer als der des Anti-Markers und unabhängig hiervon meist aber zusätzlich hierzu auch noch der Anteil des zweiten Markers um 15% bzw. 25 % höher als der des zweiten Anti-Markers). In den meisten Fällen gilt zudem außerdem, dass $\overline{n_\gamma} > 1{,}25 * \overline{n_\delta}$ und $\overline{n_\gamma} > 1{,}25 * \overline{n_\varepsilon}$

Gemäß einer weiteren Ausführungsform können außerdem auch die Produktverbindungen $\kappa_x$ und $\lambda_x$ (gemäß der vorstehenden ersten Alternative zur Bestimmung des erhöhten Anteils von Kohlenwasserstoffen mit mittleren Kettenlängen) erfasst werden, wobei $\kappa_x$ für die Produktverbindungen mit y+1 Kohlenstoffatomen und $\lambda_x$ für die Produktverbindungen mit genau y Kohlenstoffatomen steht. Auch hierbei werden wiederum nur die unverzweigten gesättigten aliphatischen Produktverbindungen und gegebenenfalls gemäß vorstehender Vorschrift auch die einfach ungesättigten unverzweigten aliphatischen Produktverbindungen erfasst. Auch hieraus kann wieder der gemittelte Anteil $\overline{n_\kappa}$ bzw. $\overline{n_\lambda}$ ermittelt werden. Auch für diese Produktverbindungen gilt dann, dass $\overline{n_\kappa} > \overline{n_\delta}$ und zusätzlich $\overline{n_\lambda} > \overline{n_\delta}$. In den meisten Fällen ist aber der Anteil $\overline{n_\lambda}$ kleiner als $\overline{n_\beta}$ und häufig zusätzlich auch noch der Anteil $\overline{n_\kappa} < \overline{n_\gamma}$. Oft gilt zusätzlich unabhängig hiervon auch, dass $\overline{n_\kappa} < \overline{n_\beta}$ und oft auch noch dass $\overline{n_\kappa} < \overline{n_\gamma}$. Mit anderen Worten ist häufig zu beobachten, dass der Anteil der Produktverbindungen $\beta$ größer ist als der der Produktverbindungen $\gamma$ und ferner auch meist größer ist als der der Produktverbindungen $\kappa$ und der der Produktverbindungen $\lambda$. Abhängig vom Ausgangsmaterial kann gegebenenfalls aber auch der Anteil der Produktverbindungen $\kappa$, $\lambda$ und/oder $\gamma$ größer sein als die der Produktverbindungen $\beta$. Zur Charakterisierung des Produktgemischs und auch des erhöhten Anteils der mittleren Kettenlängen hat es sich aber als sinnvoll erwiesen, die Produktverbindungen $\beta$ als Marker zu wählen, da diese in der Mehrheit der Fälle den größten Anteil besitzen.

Gemäß einer weiteren Ausführungsform wird das in Schritt A) bereitgestellte Ausgangsmaterial ausgewählt aus der Gruppe bestehend aus Fetten, Ölen, Fettsäuren, Fettsäureestern, Tallölen, Monoglyceriden, Diglyceriden, Alkoholen und Polyolen sowie Gemischen der vorstehenden Materialien untereinander sowie die Mischung der genannten Materialien mit anderen Stoffen, wobei aber die vorgenannten Materialien als Hauptkomponente (also mit mehr als 50 Gew.-%) vorliegen. Die Ausgangsmaterialien können ferner Terpene, wie beispielsweise Carotinoide oder Squalen enthalten. Als Ausgangsmaterialien eignen sich hier insbesondere biobasierte Ausgangsmaterialien, vorzugsweise biobasierte Fette und Öle, die den geforderten Anteil von Olefinfragmenten aufweisen.

Unter Fetten und Ölen ist erfindungsgemäß dabei folgendes zu verstehen: feste, halbfeste oder flüssige Produkte des Pflanzen- oder Tierkörpers, die zudem meist mehr oder weniger viskos sind, die chemisch im Wesentlichen aus Gemischen Triglyceriden von Fettsäuren mit mindestens 14 Kohlenstoffatomen sowie geringen Anteilen von weiteren Stoffen bestehen. Im Wesentlichen bedeutet hierbei, dass zumindest 75 Gew.-%, häufig aber zumindest 90 Gew.-%, beispielsweise zumindest 95 Gew.-% vorliegen. Neben den Triglyceriden können beispielsweise Acyllipide (z.B. Sterolester) und unversalzbare Bestandteile vorliegen. Daneben können auch Fremdbestandteile (Xenobiotika) wie Mineralöle, Weichmacher und/oder Biozide, die sich aufgrund ihres lipophilen Charakters im Fett anreichern vorliegen. Fette sind bei 20°C feste oder halbfeste Stoffe, Öle sind bei diesen Temperaturen flüssig. Zusammen mit den "fettähnlichen Verbindungen" (früher Lipoide) werden die Fette und Öle zur Gruppe der Lipide zusammengefasst.

Unter einer Fettsäure wird erfindungsgemäß jede aliphatische, gesättigte und ungesättigte Carbonsäure verstanden, die im Einzelfall auch verzweigt sein kann.

Gemäß einer Ausführungsform können die Ausgangsmaterialien, die in Schritt A) bereitgestellt werden, zumindest teilweise oder vollständig aus Abfallstoffen, industriellen Koppelprodukten und oder aus nachwachsenden Rohstoffen bestehen. Alternativ können die Ausgangsmaterialien auch aus den vorgenannten Stoffen hergestellt werden. In vielen Fällen werden Ausgangsmaterialien, die aus nachwachsenden Rohstoffen hergestellt sind oder die nachwachsenden Rohstoffe selbst sind Verwendung finden, da hierbei eine gezielte Züchtung oder Auswahl von Stoffen erfolgen kann, welche den geforderten Anteil von Olefinfragmenten aufweisen.

Gemäß einer Ausführungsform kann daher das Ausgangsmaterial ausgewählt sein aus Algen, Blaualgen, Bakterien und/oder anderen Pflanzen mit einem hohen Anteil einfach ungesättigter Fettsäuren. Alternativ können die Ausgangsmaterialien auch hergestellt werden aus Algen, Blaualgen, Bakterien und/oder Pflanzen mit einem hohen Anteil einfach ungesättigter Fettsäuren.

Ein besonders hoher Anteil von Olefinfragmenten, die der vorstehenden Maßgabe entsprechen, kann entweder durch Züchtung der Pflanzen oder Verwendung spezieller Pflanzensorten erreicht werden; alternativ kann aber auch eine gezielte genetische Modifikation von Algen, Blaualgen, Bakterien oder anderen Pflanzen erfolgen. Dann kann nicht nur der erfindungsgemäß geforderte Anteil von 50 Gew.-% ungesättigter sauerstoffhaltiger Kohlenwasserstoff-Verbindungen erreicht werden, sondern sogar ein Anteil von zumindest 75 Gew.-%, beispielsweise mindestens 90 Gew.-%

oder im Einzelfall sogar mindestens 95 Gew.-%.

Gemäß einer Ausführungsform beträgt der Anteil von einer, zwei oder drei Säuren ausgewählt aus der Gruppe bestehen aus Ölsäure, Palmitoleinsäure, Erucasäure bezogen auf alle im Ausgangsmaterial enthaltenen Fettsäuren bzw. Fettsäureester, zumindest 50 mol-%, insbesondere zumindest 75 mol-%. Gegebenenfalls können in den zumindest 50 mol-%, insbesondere zumindest 75 mol-%, alternativ oder zusätzlich auch eine oder mehrere Säuren enthalten sein, die Ölsäure, Palmitoleinsäure, Erucasäure beim Erhitzen während Schritt B) bilden (beispielsweise Ricinolsäure).

Die genannten Fettsäuren sind in hohen Mengen auch in bestimmten bereits erhältlichen Ausgangsmaterialien enthalten. So enthält High-Oleic-Sonnenblumenöl einen sehr hohen Anteil Ölsäure, bestimmte Rapssorten einen sehr hohen Anteil Erucasäure und zusätzlich Ölsäure (beispielsweise der bis Anfang der 1970iger Jahre verwendete Raps, der aufgrund seines hohen Anteils an Erucasäure kaum als Lebens- und Futtermittel verwendet werden konnte) und Cyanobakterien einen sehr hohen Anteil Palmitoleinsäure und/oder korrespondierenden Fettsäure mit 16 C-Atomen, die die cis-Doppelbindung in Position 7 aufweist. Schließlich ist auch noch High-Oleic-Distelöl mit einem Anteil einfach ungesättigter Fettsäuren, der über 70% liegt, zu nennen.

Gemäß einer weiteren Ausführungsform wird das erfindungsgemäße Verfahren so durchgeführt, dass dem in Schritt A) eingesetzten Ausgangsmaterial zuvor schon Wertstoffe entzogen wurden. Das Ausgangsmaterial ist dann also insbesondere aus Abfallstoffen, industriellen Koppelprodukten und/oder aus nachwachsenden Rohstoffen aufbereitetes Material und wird nicht direkt eingesetzt. Als Wertstoffe können dabei insbesondere mehrfach ungesättigte Fettsäuren und/oder Fettbegleitstoffe abgetrennt werden. Wie vorstehend bereits erläutert, kann in den meisten Fällen aus mehrfach ungesättigten Fettsäuren nicht das gewünschte Produktspektrum mit einem erhöhten Anteil von Kohlenwasserstoffen mit mittleren Kettenlängen erreicht werden, da diese in Schritt B) unerwünschte Nebenreaktionen eingehen. Sie können daher beispielsweise für die Nahrungsmittelproduktion aus den Ausgangsmaterialien abgetrennt werden. Eine Abtrennung kann beispielsweise mittels des in der EP 2 072 102 B1 beschriebenen Verfahrens erfolgen. Dieses Verfahren eignet sich insbesondere aber zur Abtrennung von Fettbegleitstoffen, also Stoffen, die in geringer Konzentration auftreten können, aber einen hohen Marktwert in Reinform besitzen. Als wichtige Vertreter von Fettbegleitstoffen sind insbesondere Tocochromanole, Carotinoide, Sterole, Lecithin und Glucosinolate zu nennen. Bezüglich weiterer Definitionen von Fettbegleitstoffen und eines möglichen Verfahrens zu deren Abtrennung wird vollumfänglich auf die EP 2 072 102 B1 verwiesen.

Gemäß einer weiteren Ausführungsform können als poröse Katalysatoren auf Kohlenstoffbasis erfindungsgemäß insbesondere Katalysatoren eingesetzt werden, die ausgewählt sind aus der Gruppe bestehend aus Aktivkohlen, Kohlenstoffmolekularsieben, Aktivkoksen, Carbon Nanotubes und Gemischen dieser Stoffe untereinander oder Gemischen dieser Stoffe mit anorganischen Stoffen, insbesondere Aluminiumoxiden, Zeolithen, Perovskiten, Tone (clays- z.B. Schichtsilikate, z.B. Saponite) und/oder Zinkchlorid oder auch anderen porösen oder nicht porösen Materialien. Die Aluminiumoxide, Zeolithe, Perovskite und Tone (clays) können natürlichen und/oder synthetischen Ursprungs sein. Alle vorstehenden Katalysatoren haben gemeinsam, dass sie eine große Oberfläche besitzen. Zum Teil liegen in den Katalysatoren saure Zentren vor, die für eine Spaltung von Bindungen vorteilhaft sein können.

Besonders gut kann Prozessschritt B) gesteuert werden, wenn Aktivkohlen eingesetzt werden. Hierbei ist sowohl eine Einstellung der Porengrößen und Porenverteilung sowie auch eine gezielte Dotierung oder Imprägnierung der Katalysatoroberfläche möglich, um das Crack-Verfahren gezielt zu beeinflussen. Die Porengrößen können beispielsweise durch Aktivierung der Aktivkohle vergrößert werden. So sind durch physikalische Aktivierungsmethoden (z.B. Wasserdampfaktivierung) Poren erhältlich, die durch Aufweitung von Submikroporen und sehr kleinen Mikroporen erhalten werden. Durch chemische Aktivierung können noch größere Poren (insbesondere ein größerer Anteil von Mesoporen erhalten werden. Durch die chemische bzw. physikalische Aktivierung wird ein Teil des Kohlenstoffs selektiv abgebaut, wodurch das gewünschte Porengefüge entsteht. Bei der Gasaktivierung (physikalischen Aktivierung) wird gegebenenfalls nach einem Verkohlungsprozess das Katalysator-Ausgangsmaterial bei 800-1000°C in einer Wasserdampf- und/oder Kohlendioxid-Atmosphäre aktiviert. Dabei wird ein Teil des Kohlenstoffs vergast und es entstehen Poren, die eine große innere Oberfläche bilden. Bei der chemischen Aktivierung werden z.B. Phosphorsäure, Zinkchlorid oder andere dehydratisierte Materialien verwendet. Ferner können auch Direktaktivate eingesetzt werden, bei denen im Gegensatz zur Formaktivkohle, bei der ein Kohlenstofflieferant zunächst in Pulverform mit einem Bindemittel vermengt wird, woran sich eine Formgebung und gegebenenfalls eine Trocknung, eine Carbonisierung und eine Aktivierung anschließen, aktivierte Granulate direkt aus dem Kohlenstoffträger durch Zerkleinerung und Aktivierung an einem gegebenenfalls zwischengeschalteten Carbonisierungsschritt erhalten werden. Hierbei werden mittlere Porengrößen, die zwischen denen von chemisch und physikalisch aktivierten Aktivkohlen liegen, erhalten.

Besonders geeignet für das erfindungsgemäße Verfahren sind insbesondere poröse Katalysatoren mit einem hohen Anteil an Mikroporen (Radien von 0,2 - 0,4 nm), d.h. einem Mikroporenanteil, der mit zumindest 800 $m^2$/g zur BET-Oberfläche beiträgt.

Gemäß einer Ausführungsform erfolgt eine Dotierung und/oder Imprägnierung des porösen Katalysators mit einem zweiten Katalysator. Unter Dotierung wird hierbei verstanden, dass der zweite Katalysator dem porösen Katalysator während dessen Herstellung zugesetzt wird, so dass im fertigen porösen Katalysator eine homogene Verteilung des zweiten Katalysators vorliegt. Dagegen erfolgt bei der Imprägnierung eine Behandlung des bereits fertigen porösen Katalysators mit einem Material, das den zweiten Katalysator enthält oder hieraus besteht, so dass der zweite Katalysator nur auf der Oberfläche (auch der Porenoberfläche) des porösen Katalysators vorhanden ist. Durch eine Dotierung und/oder Imprägnierung können also gezielt chemisch aktive Substanzen in das Porensystem bzw. die Matrix des Katalysators eingebracht werden, die die chemischen Reaktionen am Katalysator verändern können. Als Dotierungs- oder Imprägnierungsstoffe können insbesondere Substanzen eingebracht werden, die selbst noch einmal katalytisch wirken; alternativ können auch Substanzen eingebracht werden, die die Eigenschaften des porösen Katalysators (zum Beispiel den pH-Wert) verändern. Je nach dem verwendeten Verfahren werden dabei die physikalischen und adsorptiven Eigenschaften des porösen Katalysators ebenfalls verändert. Um Schadstoffe aus dem Ausgangsmaterial zu entfernen, können gemäß einer Ausführungsform des erfindungsgemäßen Verfahrens dem erfindungsgemäßen porösen Katalysator zweite Katalysatoren zugesetzt werden, die dazu dienen, diese Schadstoffe oder Fremdstoffe zu entfernen und/oder in abtrennbare Gase umzuwandeln. Beispielsweise können Katalysatoren, die Manganoxid enthalten, im Ausgangsmaterial enthaltenen Schwefel umwandeln. Bezüglich des Herstellungsverfahrens derart mit einem zweiten Katalysator dotierter Katalysatoren sowie möglicher zweiter Katalysatoren sei auf die WO 2007/137856 A2 verwiesen (die allerdings statt von "zweiten Katalysatoren" von Dotierstoffen spricht). Auf diese Schrift wird vollumfänglich Bezug genommen.

Gemäß einer Ausführungsform wird das erfindungsgemäße Verfahren so durchgeführt, dass die Ausgangsstoffe dem Konvertierungsreaktor in flüssiger Form zugeführt werden. Dies hat den Vorteil, dass eine energieintensive Überführung des unkonvertierten Ausgangsmaterials in die Gasphase (bzw. Dampfphase) nicht erforderlich ist. Allerdings erfordert diese Variante meistens Drücke >2000 hPa.

Gemäß einer alternativen Ausführungsform wird das Ausgangsmaterial in der Dampf- bzw. Gas-Phase in den Konvertierungsreaktor überführt. Beispielsweise ist hierzu ein kombinierter Reaktions- und Phasenübergangsapparat geeignet, in dem eine Verdampfung des Ausgangsmaterials und/oder eine Zersetzung des Ausgangsmaterials in verdampfbare Produkte und eine Verdampfung des zersetzten Ausgangsmaterials erfolgt; im Rahmen dieser Anmeldung wird dies Verdampfer genannt. In einem derartigen Verdampfer können auch bereits erste Crack-Reaktionen stattfinden, insbesondere wenn Moleküle mit großen Molekulargewichten eingesetzt werden. Beispielsweise sind hierbei Triglyceride zu nennen; auch bei unzersetzt verdampfbaren Verbindungen können aber bereits erste Crack-Prozesse erfolgen. Demzufolge kann gemäß einer Ausführungsform das Verfahren so durchgeführt werden, dass die eigentliche Crack-Reaktion erst im Konvertierungsreaktor stattfindet und im Verdampfer möglichst wenig Crack-Prozesse stattfinden. Hierzu kann der Verdampfprozess so durchgeführt werden, dass das Ausgangsmaterial vorbehandelt wird (beispielsweise indem Triglyceride zu einfachen Fettsäureestern verseift werden) oder indem das Ausgangsmaterial so langsam aufgeheizt wird, dass zwar gerade ein Verdampfen erfolgt aber noch keine Zersetzung. Ferner kann die mittlere Wandtemperatur des Verdampfers so gewählt werden, dass sie der momentanen Verdampfungs- bzw. Zersetzungstemperatur entspricht.

In einer weiteren Ausführungsform kann das erfindungsgemäße Verfahren so durchgeführt werden, dass die Kontaktierung des Ausgangsmaterials mit dem porösen Katalysator in Gegenwart von Wasser und/oder einem Wasser freisetzenden Material erfolgt. Durch den Zusatz von Wasser oder Wasser freisetzenden Materialien ist eine Verlängerung der Standzeit des Katalysators möglich. Der Zusatz von Wasser ist daher insbesondere bei kontinuierlich durchzuführenden Verfahren sinnvoll. Bevorzugt sollte mindestens so viel Wasser enthalten sein, dass - bezogen auf die zu konvertierenden Ausgangsmaterialien - 25 mindestens ein Moläquivalent Wasser (in freier Form oder in Form von Wasser freisetzenden Stoffen) zugegen ist. Ganz allgemein wird unter einem Wasser freisetzenden Material ein Stoff oder ein Stoffgemisch verstanden, das entweder gebundenes Wasser enthält, das freigesetzt werden kann oder ein Stoff oder Stoffgemisch das mittels einer chemischen Reaktion, zum Beispiel einer Kondensationsreaktion, Wasser bildet (zum Beispiel Glycerin).

Der Zusatz von Wasser kann insbesondere durch Einleiten eines Wasser- oder Dampfstromes in den Konvertierungsreaktor erfolgen. Statt Wasser können hier auch wasserhaltige Stoffe oder Stoffe die unter den im Konvertierungsreaktor herrschenden Reaktionsbedingungen Wasser abspalten, verwendet werden. Das Wasser, wasserhaltige Gemisch oder Wasser freisetzende Material kann auch den Ausgangsstoffen zugesetzt werden. Häufig ist auch in den Ausgangsstoffen bereits Wasser enthalten. Schließlich kann das Wasser oder das Wasser freisetzende Material auch dem Inertgasstrom zugesetzt werden.

Als Konvertierungsreaktor kann ein beliebiger, in geeigneter Weise heizbarer Ofen eingesetzt werden. Die Konvertierung kann kontinuierlich oder nicht kontinuierlich erfolgen. Unter kontinuierlichem Betrieb ist hierbei zu verstehen, dass die Zufuhr an Ausgangsmaterialien kontinuierlich erfolgt. Insbesondere können die Ausgangsmaterialien gasförmig bzw. dampfförmig zugegeben werden. Als Konvertierungsreaktoren kommen somit Festbettreaktoren jeglicher Bauart, Wanderbetten, Flugstromreaktoren, stationäre und zirkulierende Wirbelschichtreaktoren (inklusive Strahlmischer), Drehrostgeneratoren, Schachtöfen, Etagenöfen, Drehrohrreaktoren oder Hordenreaktoren in Betracht.

Der poröse Katalysator kann im Konvertierungsreaktor in beliebiger Weise so angeordnet sein, dass die zu konvertierenden Stoffe den Katalysator durch- oder überstreichen können. Der Kontakt der flüssigen oder dampfförmigen Ausgangsmaterialien mit dem porösen Katalysator kann auf eine beliebige geeignete Weise erfolgen, beispielsweise durch Aufsprühen von flüssigen, kalten oder erhitzten Ausgangsmaterial oder dadurch, dass ein Gasstrom mit gasförmigen beziehungsweise dampfförmigen Ausgangssubstanzen durch das Bett geleitet wird. In manchen Fällen wird auch ein fester Ausgangsstoff direkt auf den erhitzten Katalysator beziehungsweise direkt in den Konvertierungsreaktor gegeben werden können. Der Katalysator kann ebenfalls kontinuierlich oder diskontinuierlich zugegeben werden.

Zur Inertisierung des Reaktionsraums sollte dieser vorab mit einem Trägergas gespült sein. Als Trägergas kommt insbesondere ein Inertgas (wie Stickstoff oder Kohlendioxid), Wasser oder Wasserdampf oder einem $CO/CO_2$ Gemisch in Betracht.

Gemäß einer weiteren Ausführungsform wird das Verfahren bei einem Druck von zumindest 20 hPa durchgeführt. Im Regelfall ist ferner ein Druck von 20.000 hPa eine sinnvolle Obergrenze für den Druck. Häufig wird das Verfahren allerdings bei verminderten Drücken von zumindest 500 hPa durchgeführt oder bei erhöhten Drücken von bis zu 3.000 hPa (wobei sich vermindert/erhöht auf den Normaldruck von 1.013 hPa bezieht). Insbesondere hat sich eine Wahl des Drucks als geeignet erwiesen, die zwischen 700 und 1.300 hPa, beispielsweise zwischen 800 und 1200 hPa liegt.

Gemäß einer Ausführungsform wird der Einsatzmassenstrom (also der Strom in die Dampf- bzw. Gasphase überführten Ausgangsmaterials) zumindest gemäß einer, insbesondere gemäß allen drei der nachfolgenden Verhältnisse eingestellt:

- Der Einsatzmassenstrom pro Reaktionsraumleervolumen (also pro Volumen der Katalysatorschüttung, die Katalysatorfeststoff, Porenvolumen und Katalysatorzwischenräume umfasst) ist 1,5 bis 9 g/(l * min), insbesondere 2 bis 8 g/(l * min), beispielsweise 4 bis 6 g/(l * min).
- Der Einsatzmassenstrom pro Katalysatormasse ist 2 bis 30 mg/(g *min), insbesondere 6 bis 18 mg/(g *min), beispielsweise 9 bis 13,0 mg/(g *min).
- Der Einsatzmassenstrom pro Katalysatormasse [g] mal Katalysatoroberfläche [$m^2/g$] (BET-Oberfläche) ist 1 bis 20 g/(min * $m^2$), insbesondere 4-16 g/(min * $m^2$), beispielsweise 8-12 g/(min * $m^2$).

Gemäß einer Ausführungsform liegen die Reaktionstemperaturen während des Schrittes B) insbesondere bei 250-600°, beispielsweise 300-600°C, besonders bevorzugt zwischen 330 und 550°C, z.B. 350°C und 500°C, oft auch 330-450°C. Ferner wird die Temperatur im Reaktor häufig so gewählt, dass sie maximal 30°C, insbesondere maximal 15°C über der momentan vorherrschenden Verdampfungstemperatur für das Ausgangsmaterial bei dem gewählten Betriebsdruck liegt. Hierdurch wird eine hohe Flüssigproduktausbeute erreicht und eine weitere thermische Zersetzung der sekundären Crack-Produkte verhindert.

Um eine thermische Zersetzung bei Reaktionen in der Gasphase möglichst gering zu halten, kann bei Normaldruck oder unter Normaldruck gearbeitet werden. Hierzu wird der Druck so weit abgesenkt, dass die Temperaturen zwischen der Verdampfungstemperatur und 15 bzw. 30°C darüber gerade noch ein primäres und sekundäres Cracken zu beobachten ist (wobei unter dem primären Cracken die Decarboxylierung zu verstehen ist). Bezüglich einer Reaktortemperatur, die um 15 bzw. 30°C über der Verdampfungstemperatur liegt, wird also Verdampfungstemperatur erfindungsgemäß jene Temperatur definiert, bei welcher zumindest 90 Gew.-% der im Ausgangsmaterial enthaltenen Fettsäuren und Glyceride verdampfen oder unter Zersetzung in die Gasphase übergehen. Bevorzugt wird das Verfahren ferner so durchgeführt, dass möglichst wenig oder gar kein flüssiges Ausgangsmaterial bzw. flüssiges aus dem Ausgangsmaterial entstandenes Zersetzungsprodukt in den Reaktor gelangt.

Gemäß einer weiteren Ausführungsform erfolgt vor oder nach Teilschritt C) eine Hydrierung und/oder Isomerisierung der im Produktgemisch enthaltenen Kohlenwasserstoffe. Auch hierdurch kann das Produktspektrum gezielt beeinflusst werden, so dass nicht mehr ein Gemisch von gesättigten und ungesättigten oder gegebenenfalls auch von verzweigten oder unverzweigten Kohlenwasserstoffen im Produktspektrum vorliegt, sondern nur noch gesättigte Kohlenwasserstoffe und/oder ein größerer Anteil verzweigter Kohlenwasserstoffe.

Eine Hydrierung, die gleichzeitig mit Schritt B) erfolgt, ist nach dem Stand der Technik zwar möglich, würde im vorliegenden Fall aber zu einer schnellen Vergiftung der Hydrierkatalystoren führen. Daher wird erfindungsgemäß eine Hydrierung - sofern gewünscht - im Anschluss an Schritt B) bzw. C) durchgeführt. Die Hydrierung erfolgt dabei insbesondere mittels einer Reaktion mit einem Wasserstoffdonator, bei dem eine dehydrierte Form des Wasserstoffdonators entsteht (z.B. Naphthalin aus Dekalin oder Naphthalin aus Tetrahydronaphthalin). Der Wasserstoffdonator kann gasförmig oder flüssig zugegeben werden, beispielsweise durch Eindüsen von flüssigem Dekalin in das gasförmige Produktgemisch, das nach Schritt B) oder C) erhalten wurde.

Weitere geeignete Wasserstoffdonatoren sind Hydroindole (z.B. Tetrahydroindol oder Dihydroindol), Indan (das zu Inden dehydriert), Hydrochinoline oder auch aromatische Systeme mit Ethylgruppe, die durch die Wasserstoffabspaltung zu einer Vinylgruppe umgewandelt wird (z.B. Ethylbenzol oder Ethylnaphthalin).

Im Einzelfall kann auch ein Wasserstoffdonator verwendet werden, der hauptsächlich mit kurzkettigen ungesättigten Verbindungen (nicht aber nicht langkettigen ungesättigten Verbindungen) reagiert, so dass er bereits dem Ausgangsmaterial in Schritt A) zugesetzt werden kann und dann selektiv mit den Produkten des sekundären Crackens (also den Kohlenwasserstoffen mittlerer Kettenlänge) reagiert, so dass Alkane gebildet werden. Insbesondere sind derartige Wasserstoffdonatoren geeignet, um terminale Doppelbindungen zur Reaktion zu bringen. Derartige terminale Doppelbindungen können insbesondere bei α1- und α2-Spaltungen (vgl. Figur 1A, 1E und 1I) entstehen.

Gemäß einer weiteren Ausführungsform wird das erfindungsgemäße Verfahren so durchgeführt, dass eine Regenerierung des Katalysators erfolgt, insbesondere nachdem Schritt B) durchgeführt wurde. Die Katalysatorregenerierung kann hierbei im für Schritt B) verwendeten Reaktor oder in einem externen Reaktor erfolgen. Während des Schrittes B) kann es Ablagerungen an den inneren und äußeren Katalysatoroberflächen geben. Wenn diese verdampfbar sind, kann durch einfache Temperaturerhöhung bis oberhalb der Verdampfungstemperatur, gegebenenfalls unterstützt durch eine Druckabsenkung und/oder einen Trägergasstrom bereits eine Katalysatorregenerierung erzielt werden. Meist sind jedoch Ablagerungen bei technisch relevanten Temperaturen nicht verdampfbar und liegen beispielsweise in Form von Kohlenstoff vor. Kohlenstoffhaltige Katalysatoren werden daher so nachbehandelt, dass sie durch eine Reaktivierung ihre Katalysatoreigenschaften wieder erlangen. Eine derartige Reaktivierung kann gemäß dem Stand der Technik beispielsweise eine erneute Aktivierung mit Wasserdampf, Kohlendioxid und in Sonderfällen auch mit Sauerstoff sein. Dabei kann ein Inertgas (z.B. Stickstoff) als Träger- und Verdünnungsgas zusätzlich eingesetzt werden. Je nach Eigenschaften des kohlenstoffhaltigen Katalysators und nach den Reaktivierungsbedingungen können im regenerierten Katalysator Abweichungen vom Porenspektrum des frischen Katalysators erreicht werden oder auch nicht. Bei falscher Reaktionsführung kann es auch zu unerwünschten Abweichungen kommen.

Gemäß einer weiteren Ausführungsform wird das Verfahren so durchgeführt, dass in Schritt B) zumindest teilweise regenerierter Katalysator eingesetzt wird. Um einen kontinuierlichen Betrieb auch hinsichtlich des Katalysators zu gewährleisten, kann die Reaktion so durchgeführt werden, dass Schritt B) in mehreren einzeln ansteuerbaren Reaktoren abläuft, von denen mindestens einer im Betriebszustand "Reaktion" und mindestens einer im Betriebszustand "Regeneration" gefahren wird. Die Betriebsweise der einzelnen Reaktoren wird dann reihum gewechselt. Beispielsweise kann bei einer Anzahl von m Reaktoren im Verschaltungszustand 1 der Reaktor 1 auf die Betriebstemperatur aufgeheizt (oder abgekühlt) werden, in Reaktor 2 frisches, verdampftes Edukt geleitet werden, in Reaktor 3 die eigentliche Konvertierungsreaktion stattfinden und in Reaktor 4 eine Abtrennung der gebildeten Produkte und Zuführung zu einer Separiereinrichtung erfolgen. In Reaktor 5 und gegebenenfalls vorhandenen weiteren für die Regenerierung erforderlichen Reaktoren können dann die 1 bis n Regenerierungsschritte ablaufen. Nach der Regenerierung kann gegebenenfalls frischer Katalysator zugesetzt werden.

Durch eine derartige Verfahrensführung wird das frische Edukt über den frisch regenerierten Katalysator geleitet und sofern der Reaktionsschritt B) in mehreren Reaktoren stattfindet, die nahezu abreagierte Edukt-Produktmischung über einen Katalysator, der kurz vor der Regenerierung steht. Alternativ kann das Verfahren aber auch so durchgeführt werden, dass das nahezu abreagierte Edukt-Produktgemisch über den frisch regenerierten Katalysator geleitet wird und das frische Edukt über den Katalysator, der kurz vor der Regenerierung steht. Selbstverständlich ist es auch möglich im Zwischenschritt Teilströme aus den einzelnen Reaktoren abzuziehen oder in diese einzuspeisen. Zusätzlich kann auch in einem Reaktor ein Austrag des Katalysators erfolgen, so dass eine externe Regenerierung stattfindet und regenerierter oder frischer Katalysator in einen Reaktor eingetragen werden. Im Extremfall können benachbarte Reaktions- bzw. Regenerierungsschritte auch zusammenfallen, so dass die gesamte Reaktion in einem ersten und die gesamte Regenerierung in einem zweiten Reaktor durchgeführt werden.

Das erfindungsgemäße Verfahren gemäß einer oder mehrerer der vorstehend beschriebenen Ausführungsformen kann insbesondere verwendet werden, um die erhaltenen Kohlenwasserstoffe mittlerer Kettenlänge als Flugzeugtreibstoff zu verwenden oder zur Herstellung von Flugzeugtreibstoff einzusetzen. Die mit dem vorliegenden Verfahren gebildeten Kohlenwasserstoffe mittlerer Kettenlänge sind nicht nur aufgrund ihres Siedepunkts hervorragend geeignet für Flugzeugtreibstoffe (die üblicherweise einen Siedepunkt von 160-290°C besitzen), insbesondere Flugzeugtreibstoffe gemäß der gültigen Norm Jet-A1, sondern auch aufgrund der Tatsache, dass - wenn diese in der Separiereinrichtung nicht abgetrennt werden - stets auch ein gewisser Anteil von Aromaten im gebildeten Produktspektrum enthalten ist. Nach dem Stand der Technik hergestelltes Bio-Kerosin der zweiten Generation enthält zunächst keine Aromaten, da diese während des Herstellungsverfahrens hydriert werden. Für Kerosin müssen diese daher wieder zugesetzt werden. Zur Herstellung dieses Bio-Kerosins zweiter Generation werden Pflanzenöle hydriert, während im vorliegenden Verfahren bei pflanzlichen Ausgangsmaterialien keine Hydrierung stattfindet sondern vielmehr ein Crack-Prozess, so dass ein ganz anderes Produktspektrum erhalten wird. Ferner kann das Ausgangsmaterial gemäß dem vorliegenden Verfahren so gewählt werden, dass schwefelhaltige Substanzen zugegen sind. Das üblicherweise derzeit eingesetzte Kerosin enthält einen gewissen Anteil Schwefel, der als Schmierstoff dient (häufig beträgt der Schwefelanteil 3000 ppm). Wird nun bei dem erfindungsgemäßen Verfahren schwefelhaltiges Ausgangsmaterial gewählt, so kann der Schwefel beispielsweise in Form von Senfölen mitdestillieren und ist somit automatisch im Produktspektrum vorhanden.

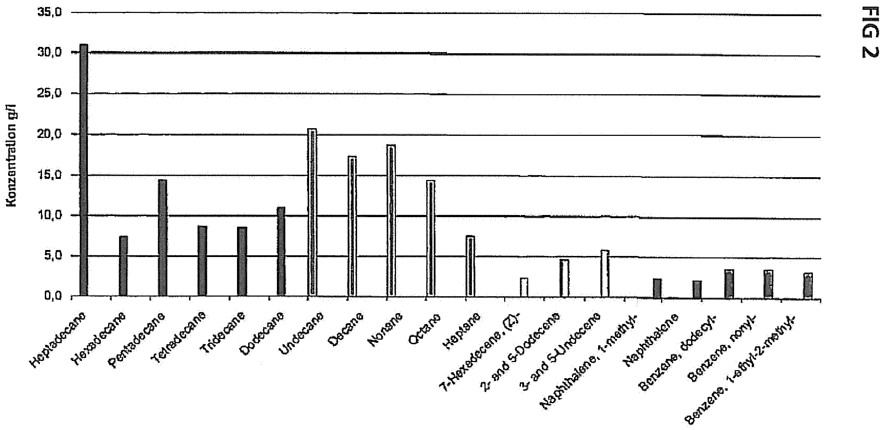

Weitere vorteilhafte Ausführungsbeispiele und Weiterbildungen der Erfindung ergeben sich im Folgenden - ohne Einschränkung der Allgemeinheit - aus den Beispielen und den Figuren 2 bis 4.

Beispiel 1 - Verwendung von Ölsäure als Ausgangsmaterial

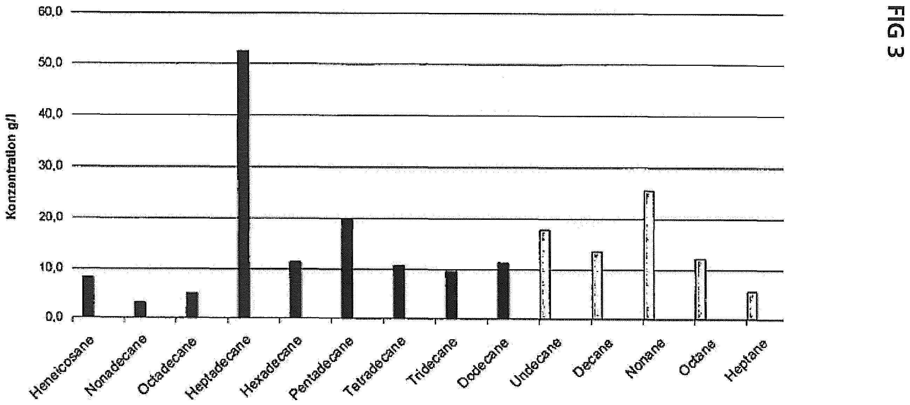

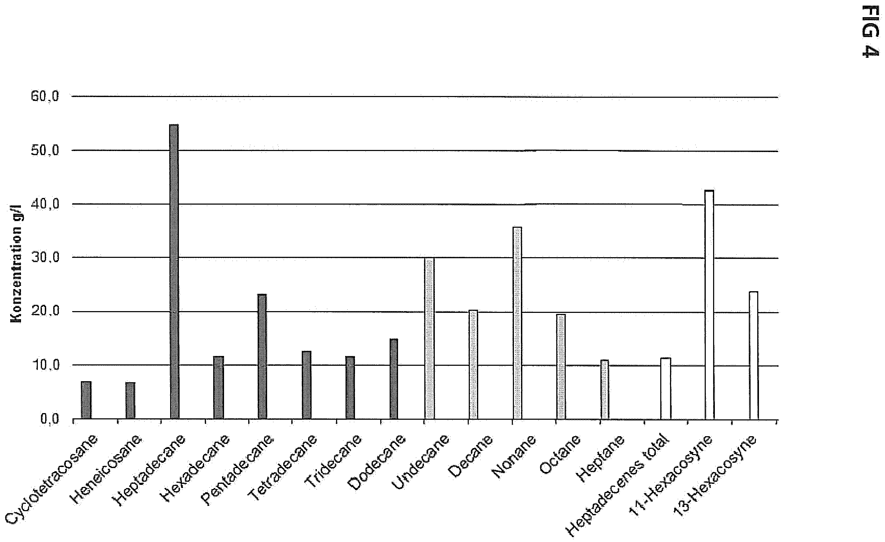

316 g Ölsäure werden gleichmäßig verteilt über 4 h mit einer Vorwärmtemperatur von 70°C in ein angewinkeltes Verdampferrohr mit einer Temperatur von 425°C gegeben. Die entstandenen Dämpfe werden in einem abwärts gerichteten Strom über einen Festbett-Katalysator geleitet. Als Festbett-Katalysator werden 80 g einer wasserdampfaktivierten Kornaktivkohle auf der Basis von Kokosnussschalen eingesetzt, mit einer spezifischen Oberfläche von 1100 $m^2/g$ (gemäß der BET-Methode) und einer Dichte von etwa 450 g/l eingesetzt(hierbei nehmen die mit BET gemessenen Poren nach der Auswertung gemäß Horvath und Kawazoe-J. Chem. Eng. Japan, 16, 6(1983), 470-475 - folgenden Oberflächenanteil ein: 0,2 bis 0,3 nm (Porenradius) -1101,3 $m^2/g$; 0,3 bis 0,4 nm - 223,7 $m^2/g$; 0,4 bis 0,5 nm - 87,3 $m^2/g$; 0,5 bis 0,6 nm - 46,5 $m^2/g$; 0,6 bis 0,7 nm - 24,1 $m^2/g$; 0,7 bis 0,8 nm - 12,1 $m^2/g$; 0,8 bis 0,9 nm - 6,7 $m^2/g$; 0,9 bis 1,0 nm - 4,1 $m^2/g$; 1,0 bis 1,1 nm - 3,9 $m^2/g$; größer 1,1 nm und kleiner 0,2 nm - nicht gemessen). Die Kornaktivkohle wurde in einem Stahlreaktor mit einer Temperatur von 450°C vorgelegt. Ferner wurden mit den Ölsäuredämpfen 100 l/h vorgewärmter Stickstoff im Gleichstrom dem Reaktionsreaktor zugeführt (Einsatzmassenstrom pro Reaktionsraumleervolumen: 5 g/(l * min)). Die entstandenen Produktgase werden einer Kühleinrichtung zugeführt und kondensiert. Tabelle 1 gibt die groben Produktfraktionen an; die Gaszusammensetzung zeigt Tabelle 2; Tabelle 3 und Figur 2 führen die Flüssigprodukte mit den höchsten Konzentrationen auf. In Tabelle 4 sind die Konzentrationen aller gesättigten und einfach ungesättigten C7-C11, C14 und C17 Kohlenwasserstoffe aufgeführt.Es handelt sich in allen Fällen (wie auch in Figur 3 und 4) jeweils um die unverzweigten Verbindungen.

Tabelle 1:

| Produkt | Anteil [Gew.-%] |
|---|---|
| Organisches Flüssigprodukt | 58 |
| Gasförmiges Produkt | 28 |
| Massenzuwachs Aktivkohle | 8 |
| Wasser | 6 |

Tabelle 2:

| Substanz | Anteil am Produktgas [mol-%] |
|---|---|
| Methan | 7,9 |
| Ethan | 3,6 |
| Ethylen | 1,7 |
| Propan | 3,4 |
| Propylen | 2,5 |
| n-Butan | 3,0 |
| iso-Pentan | 0,1 |
| n-Pentan | 2,1 |
| Heptan | 0,1 |
| Kohlendioxid | 21,1 |
| Kohlenmonoxid | 47,5 |
| Wasserstoff | 7,0 |

Tabelle 3:

| Substanz | Konzentration im organischen Flüssigprodukt [g/l], ermittelt mittels Gaschromatographie (GC) |
|---|---|
| Heptadecan | 31,0 |

(fortgesetzt)

| Substanz | Konzentration im organischen Flüssigprodukt [g/l], ermittelt mittels Gaschromatographie (GC) |
|---|---|
| Hexadecan | 7,4 |
| Pentadecan | 14,4 |
| Tetradecan | 8,7 |
| Tridecan | 8,5 |
| Dodecan | 10,9 |
| Undecan | 20,7 |
| Decan | 17,2 |
| Nonan | 18,7 |
| Octan | 14,4 |
| Heptan | 7,5 |
| (Z)-7-Hexadecen | 2,4 |
| 2- und 5-Dodecen | 4,6 |
| 3- und 5-Undecen | 5,8 |
| 1-Methyl-napthalin | 2,3 |
| Naphthalin | 2,2 |
| Dodecylbenzol | 3,6 |
| Nonyl-Benzol | 3,5 |
| 1-Ethyl-2-methyl-Benzol | 3,2 |

Tabelle 4:

| Substanz | Konzentration [Masse %] im organischen Flüssigprodukt, ermittelt mit GC | | Konzentration [Masse %] im organischen Flüssigprodukt, ermittelt mit GC |
|---|---|---|---|
| Heptadecan | 3,9 | C17 | 6,5 |
| Heptadecene | 2,6 | | |
| Tetradecan | 1,1 | C14 | 1,8 |
| Tetradecene | 0,7 | | |
| Undecan | 2,6 | C11 | 5,2 |
| Undecene | 2,6 | | |
| Decan | 2,2 | C10 | 3,7 |
| Decene | 1,5 | | |
| Nonan | 2,3 | C9 | 3,3 |
| Nonene | 1,0 | | |
| Octan | 1,8 | C8 | 2,9 |
| Octene | 1,1 | | |
| Heptan | 0,9 | C7 | 1,5 |
| Heptene | 0,6 | | |
| Alle Messungen wurden durch Mittelwerte von 12 Messungen ermittelt. | | | |

Es zeigt sich, dass bei reiner Ölsäure neben dem reinen Decarboxylierungsprodukt Heptadecan in verstärktem Ausmaß Undecan und Undecen (also die Produktverbindungen β mit y+3 = 11 Kohlenstoffatomen gebildet werden. Ferner wird relativ viel Decan und Decen gebildet (also Produktverbindung γ mit y+2 = 10 Kohlenstoffatomen). Daneben ist auch ein erhöhter Anteil von Nonan, Nonen sowie Octen und Octan zu verzeichnen (Produktverbindungen κ und λ). Im Gegenzug ist der Heptananteil relativ gering (Produktverbindungen δ mit γ-1 = 7 Kohlenstoffatomen). Ferner liegt auch ein relativ geringer Anteil von Tetradecan und Tetradecen vor (also den Produktverbindungen ε).

Beispiel 1 zeigt, dass bei einer reinen Ausgangsverbindung, also einer Verbindung, bei der lediglich eine Hauptkomponente $K_1$ zu 100 % als Olefinfragment vorliegt, verstärkt Produktkomponenten mit 11 - und daneben auch mit 10 und 9 - Kohlenstoffatomen gebildet werden, wobei durch die starke Bildung von Undecenen die $C_{11}$-Komponente besonders stark hervortritt - in Figur 2 sind hierzu die Balken für Undecan und die Undecen zusammenzurechnen und erreichen fast den Wert für das reine Decarboxylierungsprodukt durch Spalten der Bindung η, nämlich Heptadecan.

Beispiel 2 - Verwendung von High-Oleic-Sonnenblumenöl als Ausgangsmaterial

Beispiel 2 wurde durchgeführt wie Beispiel 1 mit dem Unterschied, dass statt reiner Ölsäure High-Oleic-Sonnenblumenöl verwendet wurde. Die Ölsäure stellt hier einen Anteil von mehr als 80 mol-% der vorliegenden Fettsäure; auch hier ist nur das zur Ölsäure korrespondierende Olefinfragment als Hauptfragment $K_1$ vorhanden. Weitere Olefinfragmente mit einem Anteil von zumindest 5 mol-% am Gemisch der Olefinfragmente liegen hier nicht vor. Die Flüssigproduktausbeute beträgt 62 Gew.-%.

Wie schon in Beispiel 1, zeigt sich auch hier (vgl. Figur 3), dass der Anteil von Undecan und Decan deutlich gegenüber dem Anteil von Heptan und Tetradecan erhöht ist. Aufgrund des gewählten Ausgangsmaterials zeigt sich hier auch ein deutlich erhöhter Peak für Nonan. In Tabelle 5 sind die mit GC ermittelten Konzentrationen der gesättigten und einfach ungesättigten C7-C 11, C14 und C17 Kohlenwasserstoffe aufgeführt.

Tabelle 5:

| Substanz | Konz. [Masse %] im organischen Flüssigprodukt, | | Konz. [Masse %] im organischen Flüssigprodukt |
|---|---|---|---|
| Heptadecan | 6,6 | C17 | 9,8 |
| Heptadecene | 3,2 | | |
| Tetradecan | 1,3 | C14 | 1,8 |
| Tetradecene | 0,2 | | |
| Undecan | 2,2 | C11 | 3,3 |
| Undecene | 1,1 | | |
| Decan | 1,7 | C10 | 2,2 |
| Decene | 0,5 | | |
| Nonan | 3,2 | C9 | 3,8 |
| Nonene | 0,6 | | |
| Octan | 1,5 | C8 | 2,0 |
| Octene | 0,5 | | |
| Heptan | 0,7 | C7 | 1,0 |
| Heptene | 0,3 | | |
| Alle Messungen wurden durch Mittelwerte von 12 Messungen ermittelt. | | | |

Beispiel 3 - Verwendung von High-Oleic-Distelöl als Ausgangsmaterial

Beispiel 3 wurde durchgeführt wie Beispiel 1 mit dem Unterschied, dass statt reiner Ölsäure High-Oleic-Distelöl und ein anderer Katalysator verwendet wurden. Das High-Oleic-Distelöl weist einen Anteil an Ölsäure stellt hier von mindestens70 Gew.-% auf; der Anteil einfach ungesättigter Fettsäuren beträgt 75 Gew.-%. Auch hier ist nur das zur Ölsäure korrespondierende Olefinfragment als Hauptfragment $K_1$ vorhanden. Weitere Olefinfragmente mit einem Anteil von zumindest 5 mol-% am Gemisch der Olefinfragmente liegen hier nicht vor.

# EP 2 766 456 B1

Als Festbett-Katalysator werden 80 g einer wasserdampfaktivierter Formaktivkohle auf der Basis von Holzkohle eingesetzt, die eine spezifische Oberfläche von 1250 $m^2$/g aufweist (gemäß der BET-Methode), eine Rütteldichte von 430 g/l (ermittelt in Anlehnung an Din EN ISO 787-11) und die spezifische kumulative Oberfläche der Mirkoproen gemäß Horvath und Kawazoe 1420 $m^2$/g.

Die Flüssigproduktausbeute beträgt 58 Gew.-%.

Wie schon in Beispiel 1 und 2, zeigt sich wieder (vgl. Figur 4), dass der Anteil von Undecan und Decan deutlich gegenüber dem Anteil von Heptan und Tetradecan erhöht ist. Aufgrund des gewählten Ausgangsmaterials zeigt sich hier auch ein deutlich erhöhter Peak für Nonan. In Tabelle 6 sind die mit GC ermittelten Konzentrationen der gesättigten Kohlenwasserstoffe mit 7 bis 11 sowie mit 14 Kohlenstoffatomen aufgeführt. Die Konzentrationen der korrespondierenden einfach ungesättigten Kohlenwasserstoffe wurden nicht bestimmt, sie liegen aber in jedem Fall jeweils unter 0,8 Masse %.

Tabelle 6:

| Substanz | Konz. [Masse %] im organischen Flüssigprodukt, | Substanz | Konz. [Masse %] im organischen Flüssigprodukt |
|---|---|---|---|
| Heptadecan | 6,8 | Decan | 2,5 |
| Heptadecene | 1,4 | Nonan | 4,5 |
| Tetradecan | 1,6 | Octan | 2,4 |
| Undecan | 3,8 | Heptan | 1,4 |

Beispiel 4 - Vergleichsbeispiel

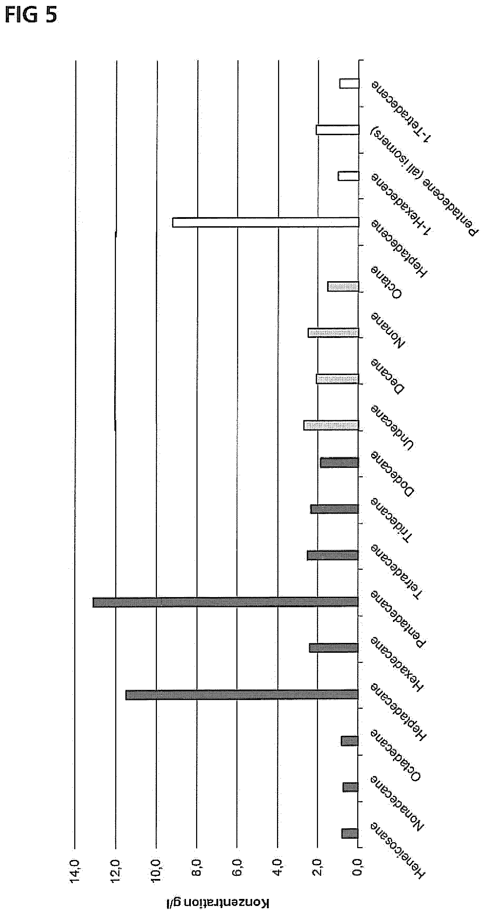

300 g gemischtes Altfett wurden gleichmäßig verteilt über 6h in ein abgewinkeltes Verdampferrohr gegeben. Das Altfett enthielt hohe Anteile von Stearinsäure und Palmitinsäure und nicht signifikante Anteile an Ölsäure .Es wurden 65 g des Katalysators aus Beispiel 1 verwendet. Alle anderen Versuchsbedingungen waren wie bei Beispiel 1. Die Ausbeute an organischer Flüssigphase betrug hier 55 Gew.-%. Figur 5 zeigt das erhaltene Produktspektrum.

Es zeigt sich, dass hier keine verstärkte Bildung von Undecan und Decan erfolgt. Der Anteil des Tetradecans (also des "Anti-Markers") ist etwa gleich hoch wie der des Undecans.

## Patentansprüche

1. Verfahren zur Gewinnung von reinen Kohlenwasserstoffen aus der Gruppe von Verbindungen mit 8 bis 16 Kohlenstoffatomen, insbesondere aus der Gruppe von Verbindungen mit 9 bis 15 Kohlenstoffatomen, oder von Kohlenwasserstoffgemischen mit einem erhöhten Anteil von Kohlenwasserstoffen mit mittleren Kettenlängen, die ausgewählt sind aus der Gruppe von Verbindungen mit 8 bis 16 Kohlenstoffatomen, insbesondere aus der Gruppe von Verbindungen mit 9 bis 15 Kohlenstoffatomen, umfassend folgende Schritte:

A) Bereitstellung eines Ausgangsmaterials, das mindestens 50 Gew.-% ungesättigte sauerstoffhaltige Kohlenwasserstoffverbindungen enthält, wobei die ungesättigten sauerstoffhaltigen Kohlenwasserstoffverbindungen zumindest teilweise einfach ungesättigte Olefinfragmente enthalten, die ausgewählt sind aus Fragmenten mit mindestens 14 Kohlenstoffatomen, insbesondere mit 16 bis 22 Kohlenstoffatomen, die der Formel $-C_1-C_xH_{2x}-CH=CH-C_yH_{2y+1}$ entsprechen, worin

- x und y ganze Zahlen sind, wobei x größer 1 ist und y größer 0 und
- das Kohlenstoffatom $C_1$ mit substituierten oder unsubstituierten Heteroatomen und/oder Wasserstoff abgesättigt ist,

wobei die Olefinfragmente eine Hauptkomponente $K_1$ mit einem Anteil von $k_1$ mol-% und gegebenenfalls weitere Komponenten $K_2 - K_x$ mit einem Anteil von $k_2 - k_x$ mol-% umfasst, der zumindest 5 mol-% beträgt, und Bereitstellung eines porösen Katalysators auf Kohlenstoff-Basis mit einem Mikroporen-Anteil, der mit zumindest 800 $m^2$/g zur BET-Oberfläche beiträgt;

B) Kontaktieren des Ausgangsmaterials in Abwesenheit von Sauerstoff bei einem Druck von 20 bis 20000 hPa und bei einer Temperatur von 200-800°C, bevorzugt 300-600 °C, insbesondere 350-550°C, in einem Konvertierungsreaktor mit dem porösen Katalysator, wobei ein kohlenwasserstoffhaltiges Produktgemisch entsteht, das einen erhöhten Anteil von Produktverbindungen an Kohlenwasserstoffen mit mittleren Kettenlängen enthält, wobei der erhöhte Anteil detektiert wird, indem

    a) für die Hauptkomponente $K_1$ und jede weitere Komponente $K_2$ - $K_x$

        - die unverzweigten und gesättigten oder einfach ungesättigten aliphatischen Produktverbindungen $\beta_x$ mit y+3 Kohlenstoffatomen,
        - die unverzweigten und gesättigten oder einfach ungesättigten aliphatischen Produktverbindungen $\delta_x$ mit y-1 Kohlenstoffatomen ermittelt werden,

    b) für alle Produktverbindungen $\beta_x$ und $\delta_x$ ihre im Produktgemisch vorliegenden Anteile $n_{\beta x}$ und $n_{\delta x}$ ermittelt werden,
    c) für die Produktverbindungen $\beta_x$ ihr gemittelter Anteil $\overline{n_\beta}$ und für die Produktverbindungen $\delta_x$ ihr gemittelter

    Anteil $\overline{n_\delta}$ gemäß der Formel $\overline{n} = \sum_{i=1}^{x} k_i * n_i$ berechnet wird,

    d) wobei ein erhöhter Anteil von Produktverbindungen an Kohlenwasserstoffen mit mittleren Kettenlängen vorliegt, wenn gilt, dass $\overline{n_\beta}$ > 1,15 * $\overline{n_\delta}$ und bevorzugt gilt, dass $\overline{n_\beta}$ > 1,5 * $\overline{n_\delta}$;

C) Auffangen des kohlenwasserstoffhaltigen Produktgemischs und Zuführung des Produktgemischs zu einer Separiereinrichtung, in der eine Produktauftrennung erfolgt.

**2.** Verfahren nach dem vorhergehenden Anspruch, wobei
die Olefinfragmente einen Anteil größer 75 mol-% an Komponenten K aufweisen, für die gilt, dass x > 4 ist, und zusätzlich
in den Schritten B)a), B)b) und B)c) für die Hauptkomponente $K_1$ und jede weitere Komponente $K_2$ - $K_x$ mit x > 4 auch die unverzweigten und gesättigten oder einfach ungesättigten aliphatischen Produktverbindungen $\varepsilon_x$ mit y+6 Kohlenstoffatomen, deren Anteil $n_{\varepsilon x}$ und der gemittelte Anteil $\overline{n_\varepsilon}$ ermittelt werden, und zusätzlich gilt, dass $\overline{n_\beta}$ > $\overline{n_\varepsilon}$, insbesondere $\overline{n_\beta}$ > 1,15* $\overline{n_\varepsilon}$, bevorzugt $\overline{n_\beta}$ > 1,25* $\overline{n_\varepsilon}$.

**3.** Verfahren nach einem der vorhergehenden Ansprüche, wobei
in den Schritten B)a), B)b) und B)c) für die Hauptkomponente $K_1$ und jede weitere Komponente $K_2$ - $K_x$ zusätzlich die unverzweigten und gesättigten oder einfach ungesättigten aliphatischen Produktverbindungen $\gamma_x$ mit y+2 Kohlenstoffatomen, deren Anteil $n_{\gamma x}$ und der gemittelte Anteil $\overline{n_\gamma}$ ermittelt werden, und zusätzlich gilt, dass $\overline{n_\gamma}$ > $\overline{n_\delta}$ und gegebenenfalls $\overline{n_\gamma}$ > $\overline{n_\varepsilon}$ und bevorzugt $\overline{n_\gamma}$ > 1,25 * $\overline{n_\delta}$ und gegebenenfalls $\overline{n_\gamma}$ > 1,25* $\overline{n_\varepsilon}$.

**4.** Verfahren nach einem der vorhergehenden Ansprüche, wobei
das Ausgangsmaterial ausgewählt wird aus der Gruppe bestehend aus Fetten, Ölen, Fettsäuren, Fettsäureestern, Tallölen, Monogalyceriden, Diglyceriden, Alkoholen und Polyolen und Gemischen hiervon.

**5.** Verfahren nach einem der vorhergehenden Ansprüche, wobei
das Ausgangsmaterial zumindest teilweise aus Abfallstoffen, industriellen Koppelprodukten und/oder aus nachwachsenden Rohstoffen besteht oder hergestellt wird.

**6.** Verfahren nach dem vorhergehenden Anspruch, wobei das Ausgangsmaterial aus Algen, Blaualgen, Bakterien und/oder Pflanzen mit einem hohen Anteil einfach ungesättigter Fettsäuren erhalten wird, wobei die Algen, Blaualgen, Bakterien oder Pflanzen genetisch modifiziert sind.

**7.** Verfahren nach dem vorhergehenden Anspruch, wobei
bezogen auf im Ausgangsmaterial enthaltene Fettsäuren der Anteil an einfach ungesättigten Fettsäuren mit mindestens 14 Kohlenstoffatomen zumindest 40 mol %, insbesondere von zumindest 50 mol %, beispielsweise von zumindest 75 mol % beträgt.

**8.** Verfahren nach einem der beiden vorhergehenden Ansprüche, wobei
bezogen auf die im Ausgangsmaterial enthaltenen Fettsäuren der Anteil an Ölsäure, Palmitoleinsäure und Erucasäure oder von Gemischen von zwei oder drei dieser Komponenten, zumindest 50 mol %, insbesondere zumindest 75 Gew.-% beträgt.

9. Verfahren nach einem der drei vorhergehenden Ansprüche, wobei aus dem Ausgangsmaterial vor Schritt A) Wertstoffe, insbesondere mehrfach ungesättigte Fettsäuren und/oder Fettbegleitstoffe, abgetrennt werden.

10. Verfahren nach einem der vorhergehenden Ansprüche, wobei
der poröse Katalysator ausgewählt wird aus der Gruppe bestehend aus Aktivkohlen, Kohlenstoffmolekularsieben, Aktivkoksen, Carbon Nanotubes, Gemischen hiervon oder Gemischen dieser Stoffe mit Aluminiumoxiden, Zeolithen, Perovskiten und/oder Zinkchlorid.

11. Verfahren nach einem der vorhergehenden Ansprüche, wobei das Ausgangsmaterial vor dem Kontaktieren mit dem porösen Katalysator einer Verdampfungseinrichtung zugeführt wird.

12. Verfahren nach einem der vorhergehenden Ansprüche, wobei
Schritt B) bei einem Druck von 500 bis 3000 hPa, insbesondere 700 bis 1300 hPa durchgeführt wird.

13. Verfahren nach einem der vorhergehenden Ansprüche, wobei
vor oder nach der Produktauftrennung eine Hydrierung und/oder Isomerisierung der im Produktgemisch enthaltenen Kohlenwasserstoffe erfolgt.

14. Verfahren nach einem der vorhergehenden Ansprüche, wobei
nach Schritt B) eine Regenerierung des Katalysators erfolgt und/oder der in Schritt A) bereitgestellte Katalysator zumindest teilweise regenerierter Katalysator ist.

## Claims

1. A method for producing pure hydrocarbons from the group of compounds having 8 to 16 carbon atoms, in particular from the group of compounds having 9 to 15 carbon atoms, or of hydrocarbon mixtures with an increased fraction of hydrocarbons having medium chain lengths which are selected from the group of compounds having 8 to 16 carbon atoms, in particular from the group of compounds having 9 to 15 carbon atoms, comprising the following steps:

A) Provision of a starting material which comprises at least 50% by weight of unsaturated oxygen-containing hydrocarbon compounds, where the unsaturated oxygen-containing hydrocarbon compounds contains at least partially monounsaturated olefin fragments which are selected from fragments having at least 14 carbon atoms, in particular having 16 to 22 carbon atoms, which correspond to the formula

$$-C_1-C_xH_{2x}-CH=CH-C_\gamma H_{2\gamma+1}$$

in which

- x and y are integers, where x is greater than 1 and y is greater than 0 and
- the carbon atom $C_1$ is saturated with substituted or unsubstituted heteroatoms and/or hydrogen,

where the olefin fragment comprises a main component $K_1$ with a fraction of $k_1$ mol% and optionally further components $K_2 - K_x$ with a fraction of $k_2 - k_x$ mol%, which is at least 5 mol%, and
provision of a porous carbon-based catalyst with a fraction of micropores which contributes with at least 800 $m^2/g$ to the BET surface area;
B) Contacting the starting material in the absence of oxygen at a pressure from 20 to 20000 hPa and at a temperature of 200-800°C, preferably 300-600°C, in particular 350-550°C, in a conversion reactor with the porous catalyst, resulting in a hydrocarbon-containing product mixture which comprises an increased fraction of product compounds of hydrocarbons with medium chain lengths,
where the increased fraction is detected by

a) ascertaining for the main component $K_1$ and each further component $K_2 - K_x$

- the unbranched and saturated or monounsaturated aliphatic product compounds $\beta_x$ with y+3 carbon atoms,

- the unbranched and saturated or monounsaturated aliphatic product compounds $\delta_x$ with y-1 carbon atoms,

b) ascertaining for all product compounds $\beta_x$ and $\delta_x$ their fractions $n_{\beta x}$ and $n_{\delta x}$ in the product mixture,

c) calculating for the product compounds $\beta_x$ their averaged fraction $\overline{n_\beta}$ and for the product compounds $\delta_x$

their average fraction $\overline{n_\delta}$ according to the formula $\overline{n} = \sum_{i=1}^{x} k_i * n_i$ ,

d) wherein an increased fraction of product compounds being hydrocarbons with medium chain lengths is present if it applies that $\overline{n_\beta} > 1{,}15 * \overline{n_\delta}$ and preferably that $\overline{n_\beta} > 1{,}5 * \overline{n_\delta}$

C) Collecting the hydrocarbon-containing product mixture and conveying the product mixture to a separating apparatus in which a product separation takes place.

2. The method according to the preceding claim, wherein
the olefin fragments contain a fraction greater than 75 mol% of components K with x being greater than 4, and wherein additionally
in steps B)a), B)b) and B)c) for the main component $K_1$ and each further component $K_2$ - $K_x$ with x > 4, also the unbranched and saturated or monounsaturated aliphatic product compounds $\varepsilon_x$ with y+6 carbon atoms their fraction $n_{\varepsilon x}$ and the averaged fraction $\overline{n_\varepsilon}$ are ascertained, and where additionally it applies that
$\overline{n_\beta} > \overline{n_\varepsilon}$ in particular $\overline{n_\beta} > 1{,}15* \overline{n_\varepsilon}$ preferably $\overline{n_\beta} > 1{,}25* \overline{n_\varepsilon}$

3. The method according to one of the preceding claims, wherein
in steps B)a), B)b) and B)c) for the main component $K_1$ and each further component $K_2$ - $K_x$ additionally the unbranched and saturated or monounsaturated aliphatic product compounds $\gamma_x$ with y+2 carbon atoms, their fraction $n_{\gamma x}$ and the averaged fraction $\overline{n_\gamma}$ are ascertained, and where additionally it applies that
$\overline{n_\gamma} > \overline{n_\delta}$ and optionally $\overline{n\gamma} > \overline{n_\varepsilon}$ and preferably $\overline{n_\gamma} > 1{,}25 * \overline{n_\delta}$ and optionally $\overline{n_\gamma} > 1{,}25* \overline{n_\varepsilon} > 1{,}25*$.

4. The method according to one of the preceding claims, wherein
the starting material is selected from the group consisting of fats, oils, fatty acids, fatty acid esters, tall oils, monoglycerides, diglycerides, alcohols and polyols and mixtures thereof.

5. The method according to one of the preceding claims, wherein
the starting material consists of or is produced at least partially from waste materials, industrial coproducts and/or from renewable raw materials.

6. The method according to the preceding claim, wherein
the starting material is obtained from algae, cyanobacteria, bacteria and/or plants with a high fraction of monounsaturated fatty acids, wherein the algae, cyanobacteria, bacteria or plants are genetically modified.

7. The method according to the preceding claim, wherein,
based on fatty acids present in the starting material, the fraction of monounsaturated fatty acids having at least 14 carbon atoms is at least 40 mol%, in particular at least 50 mol%, for example at least 75 mol%.

8. The method according to one of the two preceding claims, wherein,
based on the fatty acids present in the starting material, the fraction of oleic acid, palmitoleic acid and erucic acid or mixtures of two or three of these components, is at least 50 mol%, in particular at least 75% by weight.

9. The method according to one of the three preceding claims, wherein
before step A) products of value, in particular polyunsaturated fatty acids and/or fatty concomitants, are separated off from the starting material.

10. The method according to one of the preceding claims, wherein
the porous catalyst is selected from the group consisting of activated carbons, carbon molecular sieves, activated cokes, carbon nanotubes, mixtures thereof or mixtures of these substances with aluminium oxides, zeolites, perovskites and/or zinc chloride.

**11.** The method according to one of the preceding claims, wherein
prior to the contacting with the porous catalyst, the starting material is fed to an evaporation device.

**12.** The method according to one of the preceding claims, wherein
step B) is carried out at a pressure from 500 to 3000 hPa, in particular 700 to 1300 hPa.

**13.** The method according to one of the preceding claims, wherein
before or after product separation a hydrogenation and/or isomerization of the hydrocarbons present in the product mixture is carried out.

**14.** The method according to one of the preceding claims, wherein
after step B) a regeneration of the catalyst is carried out and/or wherein the catalyst provided in step A) is at least partially regenerated catalyst.

**Revendications**

**1.** Procédé pour l'obtention d'hydrocarbures purs choisis dans le groupe de composés ayant de 8 à 16 atomes de carbone, en particulier dans le groupe de composés ayant de 9 à 15 atomes de carbone, ou de mélanges d'hydrocarbures ayant une forte proportion d'hydrocarbures à moyennes longueurs de chaîne, qui sont choisis dans le groupe de composés ayant de 8 à 16 atomes de carbone, en particulier dans le groupe de composés ayant de 9 à 15 atomes de carbone, comprenant les étapes suivantes :

A) disposition d'un produit de départ qui contient au moins 50 % en poids de composés hydrocarbonés insaturés oxygénés, les composés hydrocarbonés insaturés oxygénés contenant au moins en partie des fragments oléfiniques mono-insaturés qui sont choisis parmi des fragments ayant au moins 14 atomes, en particulier ayant de 16 à 22 atomes de carbone, qui correspondent à la formule

$$-C_1-C_xH_{2x}-CH=CH-C_yH_{2y+1},$$

dans laquelle

- x et y sont des nombres entiers, x étant supérieur à 1 et y étant supérieur à 0 et
- l'atome de carbone $C_1$ est saturé par des hétéroatomes substitués ou non substitués et/ou des atomes d'hydrogène,

les fragments oléfiniques comprenant un composant principal $K_1$ en une proportion de $k_1$ % en moles et éventuellement d'autres composants $K_2 - K_x$ en une proportion de $k_2 - k_x$ % en moles, qui vaut au moins 5 % en moles, et disposition d'un catalyseur poreux à base de carbone, ayant une proportion de micropores qui contribue à raison d'au moins 800 m²/g à la surface BET ;
B) mise en contact du produit de départ avec le catalyseur poreux dans un réacteur de conversion, en absence d'oxygène, sous une pression de 20 à 20 000 hPa et à une température de 200-800 °C, de préférence de 300-600 °C, en particulier de 350-550 °C, de sorte qu'il en résulte un mélange de produits contenant des hydrocarbures, qui contient une proportion accrue de composés produits consistant en des hydrocarbures à moyennes longueurs de chaîne,
la proportion accrue étant détectée en

a) déterminant pour le composant principal $K_1$ et chaque autre composant $K_2 - K_x$

- les composés aliphatiques produits non ramifiés et saturés ou mono-insaturés $\beta_x$ ayant y+3 atomes de carbone,
- les composés aliphatiques produits non ramifiés et saturés ou mono-insaturés $\delta_x$ ayant y-1 atomes de carbone,

b) en déterminant pour tous les composés produits $\beta_x$ et $\delta_x$ leurs proportions $n_{\beta x}$ et $n_{\delta x}$ présentes dans le mélange de produits,
c) en calculant pour les composés produits $\beta_x$ leur proportion moyenne $\overline{n_\beta}$ et pour les composés produits

$\delta_x$ leur proportion moyenne $\overline{n_\delta}$ selon la formule $\overline{n} = \sum_{i=1}^{x} k_i * n_i$,

d) une proportion accrue de composés produits consistant en des hydrocarbures à moyennes longueurs de chaine étant présente lorsqu'il s'applique que $\overline{n_\beta} > 1{,}15^* \overline{n_\delta}$ et de préférence il s'applique que $n_\beta > 1{,}5 * \overline{n_\delta}$;

C) collecte du mélange de produits contenant des hydrocarbures et envoi du mélange de produits dans un dispositif de séparation, dans lequel a lieu la séparation des produits.

2. Procédé selon la revendication précédente, dans lequel
les fragments oléfiniques présentent une teneur de plus de 75 % en moles en composants K, pour lesquels il s'applique que x est > 4, et en outre
dans les étapes B)a), B)b) et B)c) pour le composant principal $K_1$ et chaque autre composant $K_2$ - $K_x$, où x > 4, également les composés aliphatiques produits $\varepsilon_x$ non ramifiés et saturés ou mono-insaturés, ayant y+6 atomes de carbone, on détermine leur proportion $n_{\varepsilon x}$ et la proportion moyenne $n_\varepsilon$, et en outre il s'applique que $\overline{n_\beta} > \overline{n_\varepsilon}$, en particulier $\overline{n_\beta} > 1{,}15^* \overline{n_\varepsilon}$, de préférence $\overline{n_\beta} > 1{,}25^* \overline{n_\varepsilon}$

3. Procédé selon l'une quelconque des revendications précédentes, dans lequel
dans les étapes B)a), B)b) et B)c) pour le composant principal $K_1$ et chaque autre composant $K_2$ - $K_x$, en outre les composés aliphatiques produits $\gamma_x$ non ramifiés et saturés ou mono-insaturés, ayant y+2 atomes de carbone, on détermine leur proportion $n_{\gamma x}$ et la proportion moyenne $n_\gamma$, et en outre il s'applique que $\overline{n_\gamma} > \overline{n_\delta}$ et éventuellement $\overline{n_\gamma} > \overline{n_\varepsilon}$ et de préférence $\overline{n_\gamma} > 1{,}25 > 1{,}25^* \overline{n_\delta}$ et éventuellement $\overline{n_\gamma} > 1{,}25^* \overline{n_\varepsilon}$

4. Procédé selon l'une quelconque des revendications précédentes, dans lequel le produit de départ est choisi dans le groupe constitué par des graisses, des huiles, des acides gras, des esters d'acides gras, des tallols, des monoglycérides, des diglycérides, des alcools et des polyols et des mélanges de ceux-ci.

5. Procédé selon l'une quelconque des revendications précédentes, dans lequel le produit de départ au moins en partie consiste en des ou est préparé à partir de déchets, coproduits industriels et/ou matières premières renouvelables.

6. Procédé selon la revendication précédente, dans lequel le produit de départ est obtenu à partir d'algues, d'algues bleues, de bactéries et/ou de plantes ayant une forte teneur en acides gras mono-insaturés, les algues, algues bleues, bactéries ou plantes étant génétiquement modifiées.

7. Procédé selon la revendication précédente, dans lequel la proportion d'acides gras mono-insaturés ayant au moins 14 atomes de carbone, par rapport aux acides gras contenus dans le produit de départ, est d'au moins 40 % en moles, en particulier d'au moins 50 % en moles, par exemple d'au moins 75 % en moles.

8. Procédé selon l'une quelconque des deux revendications précédentes, dans lequel la proportion d'acide oléique, d'acide palmitoléique et d'acide érucique ou de mélanges de deux ou trois de ces composants, par rapport aux acides gras contenus dans le produit de départ, est d'au moins 50 % en moles, en particulier d'au moins 75 % en moles.

9. Procédé selon l'une quelconque des trois revendications précédentes, dans lequel avant l'étape A) on sépare du produit de départ des substances de valeur, en particulier des acides gras polyinsaturés et/ou des substances graisseuses accompagnatrices.

10. Procédé selon l'une quelconque des revendications précédentes, dans lequel le catalyseur poreux est choisi dans le groupe constitué par les charbons actifs, les tamis moléculaires carbonés, les cokes actifs, les nanotubes de carbone, des mélanges de ceux-ci ou des mélanges de ces substances avec des oxydes d'aluminium, des zéolithes, des pérovskites et/ou le chlorure de zinc.

11. Procédé selon l'une quelconque des revendications précédentes, dans lequel avant la mise en contact avec le catalyseur poreux on envoie le produit de départ à un dispositif d'évaporation.

**12.** Procédé selon l'une quelconque des revendications précédentes, dans lequel on effectue l'étape B) sous une pression de 500 à 3 000 hPa, en particulier de 700 à 1 300 hPa.

**13.** Procédé selon l'une quelconque des revendications précédentes, dans lequel avant ou après la séparation des produits a lieu une hydrogénation et/ou une isomérisation des hydrocarbures contenus dans le mélange de produits.

**14.** Procédé selon l'une quelconque des revendications précédentes, dans lequel après l'étape B) a lieu une régénération du catalyseur et/ou le catalyseur disposé dans l'étape A) est au moins en partie un catalyseur régénéré.

**FIG 1A**

**FIG 1B**

**FIG 1C**

# FIG 1D

**FIG 1E**

**FIG 1F**

**FIG 1G**

## FIG 1H

β1: C11

+ C10

α1: C10

+ C11

α2: C8

+ C13

β2: C7

+ C14

**FIG 1I**

**FIG 1K**

**FIG 1L**

## FIG 1M

β1: C9

+ C6

α1: C8

+ C7

α2: C6

+ C9

β2: C5

+ C10

## FIG 2

# FIG 3

## FIG 4

## FIG 5

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 10327059 A1
- EP 1724325 A1
- EP 2072102 B1
- WO 2007137856 A2


**In der Beschreibung aufgeführte Nicht-Patentliteratur**

- **J. FU et al.** *ACS Catalysis,* 2011, vol. 1 (3), 227-231
- **HORVATH ; KAWAZOE.** *J. Chem. Eng. Japan,* 1983, vol. 16 (6), 470-475